(19)

Europäisches
Patentamt
European
Patent Office
Office européen
des brevets

(11) **EP 4 671 274 A1**

(12) **EUROPEAN PATENT APPLICATION**
published in accordance with Art. 153(4) EPC

(43) Date of publication:
**31.12.2025 Bulletin 2026/01**

(21) Application number: **24771248.2**

(22) Date of filing: **14.03.2024**

(51) International Patent Classification (IPC):
*C07K 16/24* (2006.01)    *A61P 35/00* (2006.01)
*A61P 37/00* (2006.01)    *A61K 39/00* (2006.01)

(52) Cooperative Patent Classification (CPC):
**A61K 39/00; A61P 35/00; A61P 37/00; C07K 16/24**

(86) International application number:
**PCT/KR2024/095498**

(87) International publication number:
**WO 2024/191249 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.03.2023 KR 20230033172
13.03.2024 KR 20240035471**

(71) Applicant: **Geovista Inc.
Yongin-si, Gyeonggi-do 17095 (KR)**

(72) Inventors:
 • **JI, Gilyong**
   **Seoul 03424 (KR)**
 • **AN, Gook Jun**
   **Yongin-si, Gyeonggi-do 17059 (KR)**
 • **HONG, Kwon Pyo**
   **Cheongju-si, Chungcheongbuk-do 28612 (KR)**
 • **HONG, Jeongwon**
   **Seoul 05313 (KR)**
 • **PARK, Da Yeon**
   **Seoul 08766 (KR)**
 • **KIM, Da Jung**
   **Siheung-si, Gyeonggi-do 15050 (KR)**
 • **CHOI, Gyoung Su**
   **Seoul 01614 (KR)**

(74) Representative: **Comoglio, Elena et al
Jacobacci & Partners S.p.A.
Corso Emilia 8
10152 Torino (IT)**

(54) **ANTIBODY SPECIFICALLY BINDING TO INTERLEUKIN-13, AND USE THEREOF**

(57)    According to one embodiment of the present disclosure, an antibody or an antigen-binding fragment thereof is provided. In one embodiment, an anti-interleukin-13 (anti-IL-13) antibody or an antigen-binding fragment thereof that specifically recognizes interleukin-13 (IL-13), as well as a pharmaceutical composition for treating immune-related diseases or cancer comprising the antibody, a host cell producing the antibody, and a vector comprising a nucleic acid encoding the antibody may be provided.

[Figure 9]

EP 4 671 274 A1

**Description**

[Technical Field]

The present disclosure relates to an antibody that specifically binds to canine interleukin-13 (Canis Interleukin-13; cIL-13) and the use thereof. More specifically, the present invention pertains to an anti-canine interleukin-13 (anti-IL-13) antibody or an antigen-binding fragment thereof, and to a pharmaceutical composition comprising the antibody or the antigen-binding fragment as an active ingredient.

[Background Art]

Interleukin-13 (IL-13) is a protein encoded by the IL13 gene in humans. It was first cloned in 1993, has a length of 1.4 kb, and is located on chromosome 5q31.1. The interleukin-13 has a molecular weight of 13 kDa and folds into a four $\alpha$-helix bundle. Its secondary structural features are similar to those of interleukin-4 (IL-4), sharing 25% sequence identity with IL-4. However, it is capable of signaling independently of IL-4. The interleukin-13 is known to be a cytokine secreted by T helper type 2 (Th2) cells, CD4 cells, natural killer T cells, mast cells, basophils, eosinophils, and macrophages. In addition, interleukin-13 is known to induce IgE synthesis, goblet cell hyperplasia, mucus overproduction, airway hyperresponsiveness, fibrosis, and upregulation of chitinase. Therefore, interleukin-13 is suspected to be a central mediator of physiological changes caused by allergic inflammation in various tissues.

Accordingly, despite the necessity for developing anti-interleukin-13 (anti-interleukin-13) therapeutics, the development and manufacture of such drugs can be complex and challenging due to multiple factors. For instance, as antibody drugs are highly specific, their development requires extensive knowledge of the target and the generation of highly selective antibodies. Furthermore, antibody drugs are subject to strict regulatory requirements, including the need to demonstrate safety, efficacy, and consistent quality. This necessitates extensive testing and documentation throughout the development and manufacturing process. Despite these challenges, the development and production of antibody drugs specific for the interleukin-13 target may lead to effective treatments for a variety of diseases in both humans and animals, including cancer and autoimmune disorders.

In relation to this, Korean Patent Application Publication No. 10-2019-0053184 has been disclosed.

[Detailed Description of the Invention]

[Technical Problem]

The present disclosure is directed to providing an anti-canine interleukin-13 antibody that blocks or effectively neutralizes the signaling of canine interleukin-13.

Furthermore, the present disclosure aims to provide a pharmaceutical composition comprising the anti-cIL-13 antibody, and a composition for the prevention and/or treatment of inflammatory diseases using the anti-canine interleukin-13 antibody.

The technical problems of the present disclosure are not limited to those mentioned above, and other technical problems not explicitly stated will be clearly understood by those skilled in the art from the following description.

[Solution to the Problem]

According to one embodiment of the present disclosure, there is provided an anti-canine interleukin-13 (anti-canis Interleukin-13) antibody or an antigen-binding fragment thereof that specifically recognizes canine interleukin-13 (cIL-13).

In one embodiment of the present disclosure, the anti-canine interleukin-13 antibody may be chimeric.

In another embodiment of the present disclosure, the anti-canine interleukin-13 antibody may be a caninized antibody.

In another embodiment of the present disclosure, the anti-canine interleukin-13 antibody may be a monoclonal antibody.

In another embodiment of the present disclosure, the anti-canine interleukin-13 antibody or its antigen-binding fragment may comprise a heavy chain constant region derived from canine immunoglobulin G (IgG) A, B, C, or D-type.

In another embodiment of the present disclosure, the anti-canine interleukin-13 antibody or its antigen-binding fragment may comprise a light chain constant region derived from canine kappa or lambda chains.

In another embodiment of the present disclosure, the amino acid sequence of the light chain variable region of the anti-canine interleukin-13 antibody or its antigen-binding fragment may be selected from SEQ ID NOs: 12, 23, 24, 25, 26, 27, and 28.

In another embodiment of the present disclosure, the amino acid sequence of the light chain of the anti-canine interleukin-13 antibody or its antigen-binding fragment may be selected from SEQ ID NOs: 30, 32, 34, 36, 38, 40, 42, 44, 46, 48, 50, 52, 54, 56, 58, 60, 62, 64, 66, 68, 70, 72, 74, 76, and 78.

In another embodiment of the present disclosure, the amino acid sequence of the heavy chain variable region of the anti-canine interleukin-13 antibody or antigen-binding fragment may be selected from SEQ ID NOs: 10, 17, 18, 19, 20, 21, and 22.

In another embodiment of the present disclosure, the amino acid sequence of the heavy chain of the anti-canine interleukin-13 antibody or antigen-binding fragment may be selected from SEQ ID NOs: 29, 31, 33, 35, 37, 39, 41, 43, 45, 47, 49, 51, 53, 55, 57, 59, 61, 63, 65, 67, 69, 71, 73, 75, and 77.

In another embodiment of the present disclosure, the anti-canine interleukin-13 antibody or its antigen-binding fragment may exhibit at least one characteristic selected from inhibition of proliferation of TF-1 cell line or inhibition of STAT6 phosphorylation.

In another embodiment of the present disclosure, the anti-canine interleukin-13 antibody or its antigen-binding fragment may comprise at least one of a heavy chain having the amino acid sequence of SEQ ID NO: 69 or 71 or a variant thereof, and a light chain having the amino acid sequence of SEQ ID NO: 70 or 72 or a variant thereof.

In another embodiment of the present disclosure, the anti-canine interleukin-13 antibody or its antigen-binding fragment may comprise an antibody consisting of two light chains and two heavy chains.

In another embodiment of the present disclosure, the anti-canine interleukin-13 antibody or its antigen-binding fragment may include Fab fragment, Fv fragment, diabody, triabody, tetrabody, or a fragment of at least one foregoing.

In another embodiment of the present disclosure, the anti-canine interleukin-13 antibody or its antigen-binding fragment may be used for the prevention or treatment of diseases in animals.

In another embodiment of the present disclosure, the anti-canine interleukin-13 antibody or its antigen-binding fragment may have a use for the prevention or treatment of at least one disease selected from the group consisting of inflammatory diseases, immune diseases, autoimmune diseases, atopic dermatitis, and cancer in animals including dogs.

In another embodiment of the present disclosure, there is provided a pharmaceutical composition for the treatment of immune diseases or cancer, comprising the antibody of the present disclosure and a pharmaceutically acceptable carrier.

In another embodiment of the present disclosure, the pharmaceutical composition for the treatment of immune diseases or cancer, comprising the antibody of the present disclosure and a pharmaceutically acceptable carrier may be formulated as an injectable preparation.

In another embodiment of the present disclosure, there is provided a host cell that produces the antibody of the present disclosure.

In another embodiment of the present disclosure, the host cell that produces the antibody of the present disclosure may be selected from the group consisting of bacteria including Escherichia coli, yeast, CHO cells, and HEK cells.

In another embodiment of the present disclosure, the host cell that produces the antibody of the present disclosure may comprise a gene encoding a chaperone protein or a glycosylation enzyme as a result of genetic engineering to enhance antibody expression and secretion.

In another embodiment of the present disclosure, there is provided a vector comprising a nucleic acid encoding the antibody of the present disclosure.

[Advantageous Effects of the Invention]

The present disclosure may provide a pharmaceutical composition for blocking or neutralizing interleukin-13 signaling, as well as a composition for the prevention and/or treatment of inflammatory diseases.

Meanwhile, the effects achievable by the present disclosure are not limited to those described above, and other effects not specifically mentioned will be clearly understood by those of ordinary skill in the art from the following description.

[Brief Description of the Drawings]

FIG. 1 illustrates the gene sequence of canine IL-13 optimized for CHO cells according to one embodiment of the present invention.

FIG. 2 illustrates an expression vector constructed by inserting the cloning vector (pUC-GW-Amp) of the CHO-optimized canine IL-13 gene, obtained by double digestion with XhoI and EcoRI, into the pcDNA 3.4 plasmid according to one embodiment of the present invention.

FIGS. 3a to 3c show IMAC chromatograms representing the purification results of recombinant cIL-13 protein cultured in ExpiCHO-S cells according to one embodiment of the present invention.

FIG. 4 shows the electrophoresis results of cIL-13 after treatment with PNGase, which removes N-linked glycans, according to one embodiment of the present invention.

FIG. 5 is a graph showing the TF-1 cell proliferation activity induced by purified cIL-13 according to one embodiment of the present invention.

FIG. 6 shows the results of analyzing the binding site of the anti-canine interleukin-13 monoclonal antibody using Western blotting after treatment with PNGase-F or O-glycosidase to remove N- or O-linked glycans.

FIG. 7 illustrates the codon-optimized sequences for CHO cells of the light and heavy chain variable region genes identified from the 32A10 hybridoma according to one embodiment of the present invention.

FIG. 8 is a graph showing the purity evaluation of two types of mouse/canine chimeric purified antibodies using SE-HPLC columns according to one embodiment of the present invention.

FIG. 9 is a graph evaluating the STAT-6 phosphorylation inhibitory activity of the chimeric purified antibody according to one embodiment of the present invention.

FIG. 10 illustrates the result of modeling the tertiary structure of the chimeric antibody according to one embodiment of the present invention.

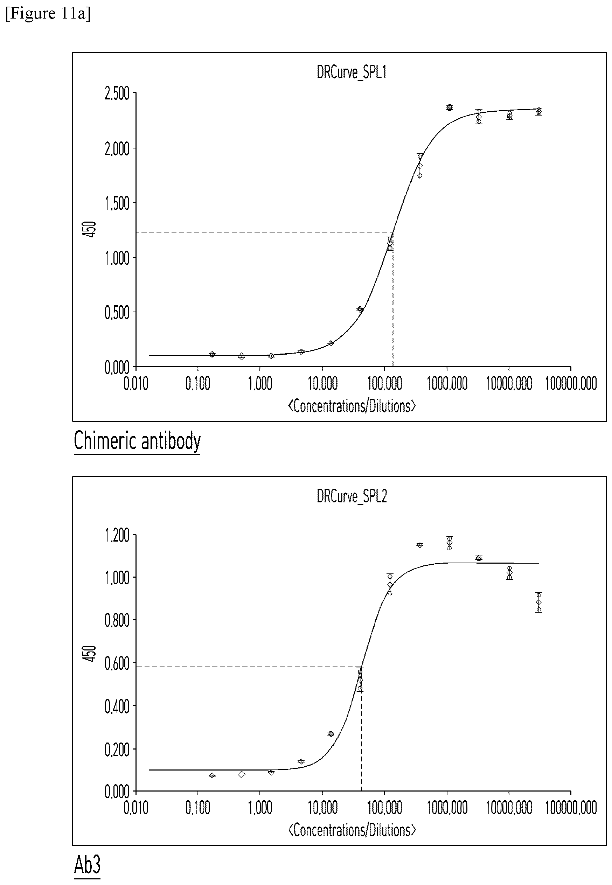

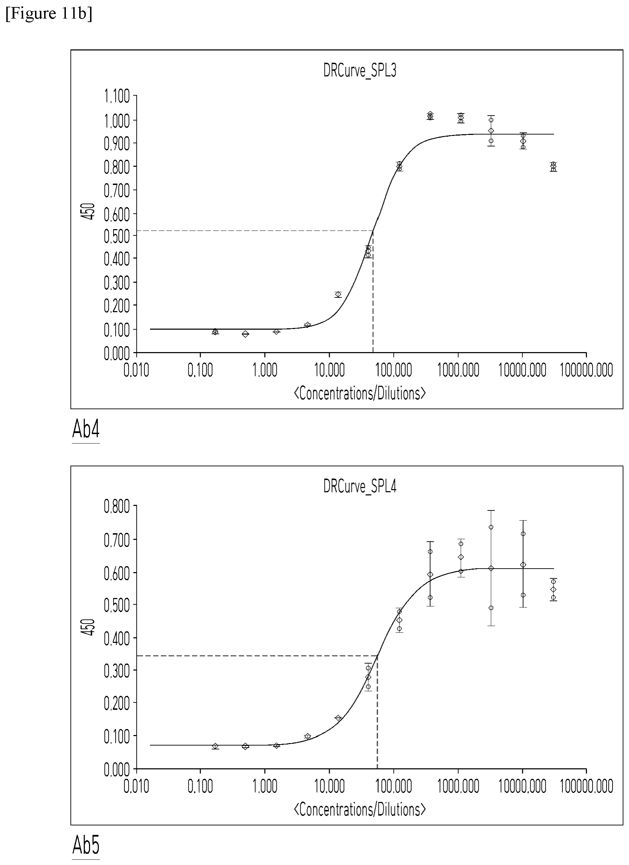

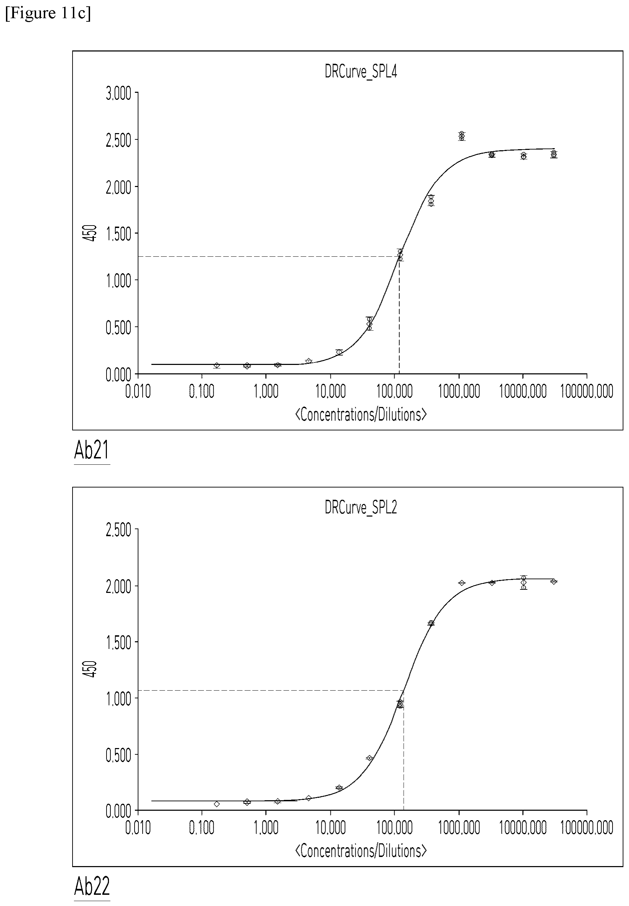

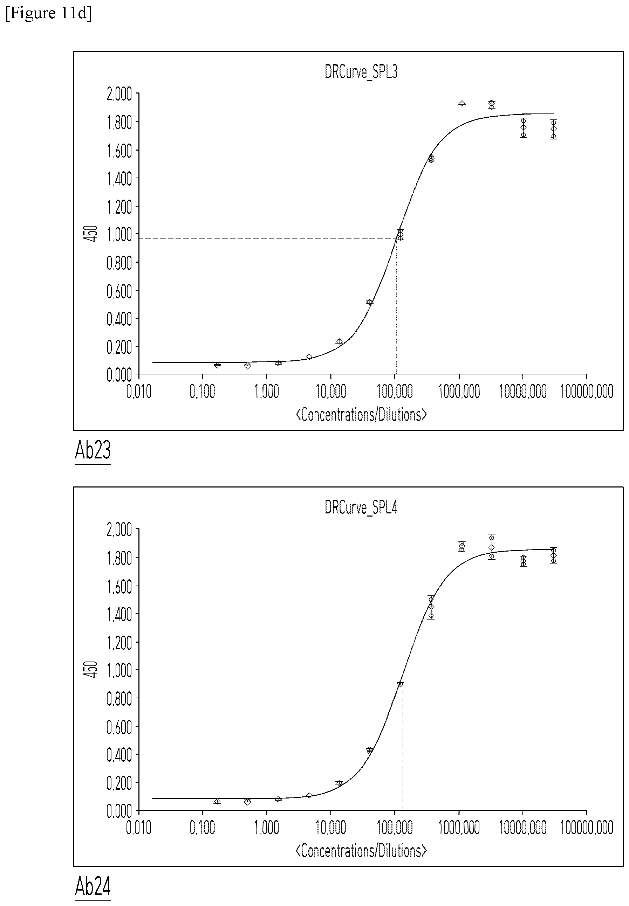

FIGS. 11a to 11d are graphs evaluating the antigen-binding affinity of the caninized antibody according to one embodiment of the present invention.

FIG. 12 is a graph evaluating the thermal stability of the caninized antibody according to one embodiment of the present invention.

FIGS. 13a to 13c are graphs evaluating the TF-1 cell inhibitory activity of the caninized antibody according to one embodiment of the present invention.

[Best Mode for Carrying Out the Invention]

Various embodiments are described with reference to the accompanying drawings. In this specification, the descriptions are provided to enhance the understanding of the present disclosure. Before describing specific details for carrying out the invention, it should be noted that elements not directly related to the technical features of the present invention may be omitted to avoid obscuring its essence. Furthermore, the terms and words used in this specification and the claims should be interpreted in accordance with the technical spirit of the invention, based on the principle that the inventor may define terms appropriately to best describe their invention.

Moreover, the term "or" is intended to be interpreted as inclusive "or" rather than exclusive "or". That is, unless otherwise specified or contextually evident, the phrase "X uses A or B" may refer to any of the natural inclusive substitutions. For example, "X uses A or B" may be applied as any of the following: X uses A ; X uses B; or X uses both A and B. Likewise, the terms "and/or" and "at least one of" should be understood to refer to and encompass all possible combinations of the listed elements. For example, "at least one of A or B" or "at least one of A and B" should be interpreted as including: A alone, B alone, or a combination of A and B.

The terms "comprises" and/or "comprising" as used herein should be interpreted as indicating the presence of the stated features and/or components, but not excluding the presence or addition of one or more other features, components, and/or groups thereof. Unless otherwise specified or contextually clear, the singular forms used herein and in the claims are intended to include the plural as well. The embodiments described herein are provided so that those skilled in the art may implement or utilize the invention. Various modifications to these embodiments will be apparent to those skilled in the art. The general principles defined herein may be applied to other embodiments without departing from the scope of the invention. Therefore, the invention is not to be limited to the embodiments disclosed herein, but should be interpreted in the broadest scope consistent with the principles and novel features described.

Interleukins are a group of cytokines primarily produced and secreted by immune cells and play a role in regulating immune responses by facilitating communication between different types of cells. Interleukins mediate signaling between leukocytes involved in immune responses and regulate proliferation, differentiation, and activation of immune cells. They influence both innate and adaptive immunity by stimulating or suppressing immune cell activity, modulating inflammation, and contributing to the resolution of immune responses. Interleukins are often released in response to immune challenges such as infection or inflammation, and their production is tightly regulated to prevent excessive immune responses that could lead to autoimmune diseases or chronic inflammation.

Dysregulation of interleukin signaling is associated with various diseases. Recombinant antibodies designed to recognize and neutralize specific interleukins or their receptors are used therapeutically to modulate their activity. For example, tocilizumab and sarilumab target interleukin-6 (IL-6); secukinumab and ixekizumab target IL-17A for the treatment of psoriasis and psoriatic arthritis; ustekinumab targets the shared p40 subunit of IL-12 and IL-23 for the treatment of psoriasis and Crohn's disease; and canakinumab targets IL-1β for treating certain auto inflammatory conditions. Dupilumab is a recombinant antibody that targets both IL-4 and IL-13 receptors and is approved for the treatment of atopic dermatitis with nasal polyps, asthma, and chronic rhinosinusitis.

Among the various subtypes of interleukins, IL-13-primarily produced by T helper 2 (Th2) cells-plays a central role in allergic inflammation by promoting IgE antibody production and enhancing eosinophil recruitment. IL-13 also affects fibroblast activation to assist in tissue remodeling and regulates immune responses by suppressing pro-inflammatory cytokines and promoting anti-inflammatory signaling. IL-13 exerts its effects by binding to a receptor complex composed of IL-13 receptor alpha 1 (IL-13Rα1) and IL-4 receptor alpha (IL-4Rα). Upon receptor binding, IL-13 initiates intracellular signaling cascades that lead to various cellular responses.

Proper regulation of IL-13 may be utilized in treating atopic dermatitis (AD), an inflammatory and immune-mediated skin condition. AD is a chronic, relapsing eczematous disorder accompanied by intense pruritus and is known to be caused by a combination of genetic predisposition, environmental factors, immunologic abnormalities, and skin barrier dysfunction. Mild cases may be managed with moisturizers, but moderate-to-severe cases may require corticosteroids, calcineurin inhibitors, or systemic immunosuppressants. Recently, systemic treatments such as dupilumab and tralokinumab-which block IL-4 and/or IL-13 signaling-have been approved in the U.S. and Europe, offering safe and effective options for patients requiring systemic therapy.

Canine atopic dermatitis (CAD) is a common allergic skin disease in dogs. CAD is characterized by chronic inflammation of the skin caused by the dog's immune system overreacting to environmental allergens. This condition resembles human atopic dermatitis or eczema. Common allergens that cause CAD include pollen, mold spores, dust mites, and specific food ingredients. Management strategies for CAD include avoiding or minimizing allergen exposure, using hypoallergenic diets, administering medications such as antihistamines or corticosteroids to relieve symptoms, and using antibiotics or antifungals for secondary infections. Immunotherapy (allergy shots) may also be recommended to desensitize the dog to specific allergens over time.

For moderate-to-severe CAD, systemic treatments are often required. Steroids and immunosuppressants like cyclosporine are widely used. Recently, Cytopoint® (lokivetmab) and Apoquel® (oclacitinib) have become available. However, because Apoquel is a JAK inhibitor that broadly suppresses cytokine signaling, it may not be suitable for an individual with hyperadrenocorticism or progressive malignant tumors and can cause side effects such as diarrhea and vomiting. Cytopoint specifically neutralizes canine IL-31 and is effective at reducing pruritus, but it is not considered a fundamental therapy. The anti-cIL-13 antibody provided in the present invention may be used as a treatment for canine atopic dermatitis, though its purposes are not limited thereto.

TF-1 cells are an immortalized cell line derived from human erythroleukemia, developed to provide a stable research model. These cells are dependent on granulocyte-macrophage colony-stimulating factor (GM-CSF) or IL-3 for growth. TF-1 cells are responsive to various cytokines and express a broad array of endogenous receptors, making them a useful model for studying cytokine signaling mechanisms.

One of the main signaling pathways induced by IL-13 involves the activation of STAT6. Members of the STAT protein family transmit signals from receptor complexes to the nucleus, thereby activating gene expression. STAT6 is activated upon cytokine exposure by Janus family tyrosine kinases (JAKs). Activated STAT6 regulates the transcription of various genes involved in immune responses, inflammation, and tissue remodeling. Many genes regulated by the IL-13/STAT6 pathway are associated with characteristics of allergic inflammation. Dysregulation of this pathway is linked to the pathogenesis of allergic diseases, and therapeutics targeting IL-13 or STAT6 are being explored for conditions such as asthma and atopic dermatitis.

Terms

Antibody

An antibody, also known as an immunoglobulin, is a large Y-shaped protein produced by the immune system in response to foreign substances known as antigens. Antibodies recognize and bind specific antigens, marking them for neutralization or destruction by other components of the immune system. A single antibody molecule consists of two heavy polypeptide chains and two light polypeptide chains. The heavy chains determine the class of the antibody (e.g., IgG, IgA, IgM) and its effector function, and each consists of a constant (HC) and variable (HV) region. The light chains are of either the kappa ($\kappa$) or lambda ($\lambda$) type and also consist of constant (LC) and variable (LV) regions. The antigen-binding site is formed by the variable regions of the heavy and light chains. Antibodies can be enzymatically fragmented into functional domains such as $F(ab')_2$, $F(ab)_2$, Fab', Fab, Fv, and scFv.

Hybridoma

Hybridoma technology, developed by Georges Köhler and César Milstein in 1975, revolutionized immunology and antibody production. The technique involves the fusion of B cells and myeloma cells to create a stable, immortalized cell line capable of producing large quantities of monoclonal antibodies. B cells produce antibodies but have limited proliferation capacity, while myeloma cells can divide indefinitely but do not produce antibodies. After fusion, the hybridomas are cultured in a selective medium that supports only fused cells. Screening is then performed to identify hybridomas producing antibodies with the desired antigen specificity. The resulting hybridomas stably produce highly specific monoclonal antibodies that recognize a single epitope on a target antigen.

Subclone

A subclone is a genetically identical subset of cells derived from a parent clone or monoclonal population. Subcloning involves isolating and culturing individual cells from the original clone to create a new, uniform population containing identical genetic material. The parent clone contains the gene(s) of interest, a specific DNA fragment or a plasmid with an insert. Subcloning may involve techniques such as cell sorting, limiting dilution, or selection based on desired genetic characteristics. The resulting subclonal colonies are expanded, and their genetic identity and properties are verified using PCR, DNA sequencing, and other analyses.

Chimeric Antibody

A chimeric antibody is a recombinant antibody in which genetic material from different species is combined to produce a hybrid molecule. While the chimeric antibody is originally developed to reduce immunogenicity in humans and retaining the antigen specificity of non-human antibodies, chimeric antibodies in this disclosure are designed for administration to dogs. The variable regions derived from mouse antibodies retain original antigen-binding specificity. The constant regions in an embodiment of this disclosure are derived from canine antibodies(and generally, from human antibodies) to reduce immunogenicity, i.e., the immune response against the antibodies. The constant regions are responsible for effector functions and interactions with other components of the immune system.

Complementarity Determining Region (CDR)

The complementarity determining regions are specific amino acid sequences located in the variable domains of an antibody's heavy and light chains. These regions interact with epitopes on the surface of the antigen through shape complementarity, electrostatic interactions, hydrogen bonding, and van der Waals forces. The immune system generates a diverse repertoire of antibodies by varying the sequences of CDRs, enabling recognition and neutralization of a wide range of pathogens. Typically, there are three CDRs each in the variable regions of the heavy and light chains.

Framework Region

The framework region (FR) refers to the structural elements of the antibody variable domain that flank and support the CDRs. While CDRs directly contact the antigen and determine binding specificity, the framework provides the structural scaffold essential for overall stability, integrity and proper folding. In the present disclosure, canine framework residues are substituted during the caninization process to reduce immunogenicity and improve therapeutic compatibility.

Canonical Structures

Canonical structures, as defined by Chothia et al., are recurring shape or structural motifs observed in antibody variable region CDR. There are six major canonical classes, labeled A through F, characterized by specific backbone dihedral angles($\phi$ and $\psi$ angles) and hydrogen bonding patterns which define overall shapes and directions of the loops. These canonical structures provide a framework for understanding structural diversity of CDR loop and are widely used in computational modeling and antibody engineering to predict and design antibody-antigen interactions. However, canonical structures are not strictly conserved across all CDRs, and variations may arise depending on the loop sequence and context.

Anchor Residues

Anchor residues refer to specific amino acids within the CDR loops that play a key role in antigen recognition and binding. These residues typically occupy structurally central positions in the loop and make direct contact with the epitope.

Vernier Zones

Vernier zones are specific amino acid residues located within or near the CDR loops. These zones influence the structure of the CDRs and fine-tuning of antigen recognition. Thus, in antibody caninization, mismatches at Vernier zones between donor (e.g., murine) CDRs and recipient (e.g., canine) frameworks may compromise affinity or stability of the antibodies, and such residues are often back-mutated to the original donor amino acids.

Post-Translational Modification (PTM)

PTMs are chemical modifications that occur after a protein is synthesized from its mRNA template. In antibodies, PTMs can significantly affect structure, function, stability, and activity. Common PTMs in antibody technologies include glycosylation, disulfide bond formation, acylation of fatty acids, PEGylation, deamidation of asparagine or glutamine, methionine oxidation, isomerization of D-aspartate, N-terminal glutamine cyclization, cysteine bonding, lysine side-chain glycation, and tryptophan oxidation.

Host Cell

A host cell is a living cell that supports the replication and expression of foreign genetic material such as DNA or RNA. It contains the enzymes and ribosomes necessary for transcription and translation of foreign genes. Host cells are engineered via transformation (for prokaryotes) or transfection (for eukaryotes) to express and produce specific proteins or antigens. These proteins are then harvested and purified to produce therapeutic proteins, vaccines, or other biotechnological products. Common host cells include Escherichia coli (for bacterial expression), Saccharomyces cerevisiae (yeast) for eukaryotic expression systems, and mammalian cell lines such as HEK293(Human Embryonic Kidney 293) or CHO(Chinese Hamster Ovary) cells for more complex eukaryotic expression systems.

Vector

In molecular biology, a vector is a DNA molecule used as a vehicle to carry and deliver foreign genetic material (e.g., a gene or a DNA fragment) into a host organism. Vectors are widely used in genetic engineering, recombinant DNA technologies and other various molecular biology application fields to manipulate and research genes. Vectors are essential in enabling replication, proliferation and expression of inserted genetic materials in host cells. Common types of vectors include plasmids, bacteriophages, cosmids, artificial chromosomes (e.g., Bacterial artificial chromosomes or BACs, Yeast artificial chromosomes or YACs), and viral vectors.

The following invention will be described in greater detail in the embodiments and illustrated in the accompanying figures.

IL-13 signaling is widely recognized as a key mediator in the pathogenesis of various immune-related diseases and cancers, including atopic conditions. The inventors of the present invention developed and validated an antibody that specifically binds and neutralizes canine IL-13. First, recombinant canine IL-13 (cIL-13) was produced. Mice were injected with recombinant cIL-13 to elicit an immune response. The inventors then harvested spleens from mice with induced immune responses and prepared a cell suspension. The splenocytes were fused with myeloma cells using polyethylene glycol (PEG) to generate hybridomas. The resulting hybridoma colonies were screened via ELISA, TF-1 proliferation inhibition, and STAT6 phosphorylation inhibition. As a result, monoclonal antibody designated 32A10-#20 was produced.

Binding of 32A10-#20 to cIL-13 was confirmed even after N-glycans or O-glycans were enzymatically removed using PNGase F or O-glycosidase, indicating glycosylation-independent epitope recognition.

According to one embodiment of the invention, the gene sequence encoding 32A10-#20 was cloned from the hybridoma. Antigen specificity and biological activity of the monoclonal antibody are determined by the variable regions, including the complementarity determining regions (CDRs). Sequence analysis using three primer sets each for the heavy and light chains revealed conserved CDRs but two distinct framework region variants. The verified variable region sequences were codon-optimized for CHO(Chinese Hamster Ovary) cells. Using an expression vector, the optimized genes were transfected into ExpiCHO-S cells, cultured, and the resulting chimeric antibody was purified via column chromatography. The antibody's purity and activity were analyzed, the effective sequences were confirmed, and functional validation was performed.

The following experimental examples provide further detailed descriptions.

**Acquisition of Canine Interleukin-13 (Canis Interleukin-13)**

The canine interleukin-13 (cIL-13) described in the present disclosure consists of 131 amino acids, with a signal peptide located at the N-terminus. N-linked glycans are attached to asparagine residues at positions 38, 48, 56, and 71. Disulfide bonds are formed between cysteine residues at positions 47-75 and 63-89, contributing to the tertiary structure of the protein. The precursor gene sequence of canine interleukin-13 was obtained using the National Center for Biotechnology Information (NCBI) database (GenBank: AF244915.1). The sequence was subsequently codon-optimized for expression in the host Chinese hamster ovary (CHO) cells, excluding restriction enzyme sites required for downstream cloning steps. For example, codon optimization was performed using publicly available tools or by outsourcing to an external organization.

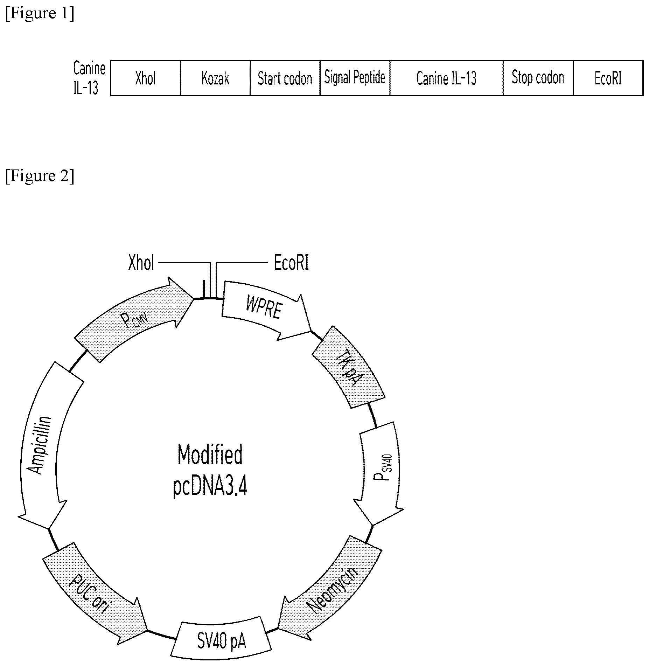

For cloning purposes, the synthetic gene construct was designed by sequentially adding the following elements: an XhoI restriction site (CTCGAG) at the 5' end, a Kozak sequence (GCCACC) required for translation, a start codon (ATG), the IL-13 coding sequence (CDS), a polyhistidine (CATCATCACCATCACCAC), a stop codon (TGA), and an EcoRI restriction site (GAATTC), as shown in FIG. 1.

The final gene sequence of canine IL-13 optimized for CHO cells is as follows:

**[Table 1]**

| CHO-Optimized Canine IL-13 Sequence |
|---|
| CTCGAGGCCACCATGGCG CTC TGG CTC ACT GTA GTA ATA GCT CTG ACA TGC TTG GGA GGC CTT GCT TCT CCC TCT CCC GTA ACC CCC TCT CCA ACA CTG AAA GAG CTC ATT GAA GAG CTC GTC AAT ATC ACA CAG AAC CAA GCA TCT CTT TGC AAT GGA AGC ATG GTC TGG AGC GTC AAT CTG ACC GCT GGC ATG TAT TGT GCC GCC CTT GAA AGC CTC ATA AAT GTA AGC GAT TGT TCA GCT ATA CAA AGA ACC CAG CGT ATG TTG AAG GCT CTT TGC AGC CAG AAA CCT GCA GCA GGT CAG ATT AGT TCT GAG AGG TCC CGC GAT ACA AAA ATC GAA GTC ATC CAG CTC GTG AAG AAT CTT CTC ACA TAC GTC CGG GGC GTA TAT CGT CAC GGA AAT TTT CGCCATCATCACCATCACCACTGAGAATTC |

**Production of Recombinant cIL-13 Protein**

Following the synthesis of CHO-optimized canine IL-13, a cloning vector (pUC-GW-Amp) was obtained. The insert DNA was isolated through double digestion with XhoI and EcoRI, purified, and subsequently ligated into a plasmid to construct an expression vector (FIG. 2). The plasmid used may be, for example, a pcDNA 3.4 vector; however, such examples are not intended to limit the scope of the present disclosure.

Thereafter, the inventors of the present invention transfected CHO cells with the constructed vector and performed transient expression in medium supplemented with L-glutamine. For instance, the ExpiCHO™ Expression System Kit (ThermoFisher, #A29133) may be used; however, such examples are not intended to limit the scope of the present disclosure.

[Table 2]

| Transient Expression | | | | | |
|---|---|---|---|---|---|
| Day | Flask No. | Viable cell density (x $10^6$ cells/ml) | Viability (%) | Volume(mL ) | Remarks |
| 0 | #1 | 5.45 | 94.0 | 22.31 | |
| | #2 | - | | 22.28 | |
| 1 | #1 | - | - | 28.46 | Feed, Enhancer added |
| | #2 | - | - | 28.43 | |
| 4 | #1 | 4.83 | 68.5 | 28.46 | |
| | #2 | 4.70 | 71.1 | 28.43 | |
| 5 | #1 | 4.48 | 60.9 | 28.46 | End of culture |
| | #2 | 4.32 | 63.2 | 28.43 | |

The culture fluid obtained after completion of the cultivation was diluted 20-fold with distilled water and sterilized by filtration to prepare the injection sample.

**Purification of Recombinant cIL-13**

In one embodiment of the present disclosure, the sample was loaded onto a resin column, and the his-tagged canine interleukin-13 (cIL-13) protein was purified using an imidazole gradient method. Although a HisTrap™ HP 5 mL column (Cytiva, #29051021) may be used, such examples are not intended to limit the scope of the present disclosure.

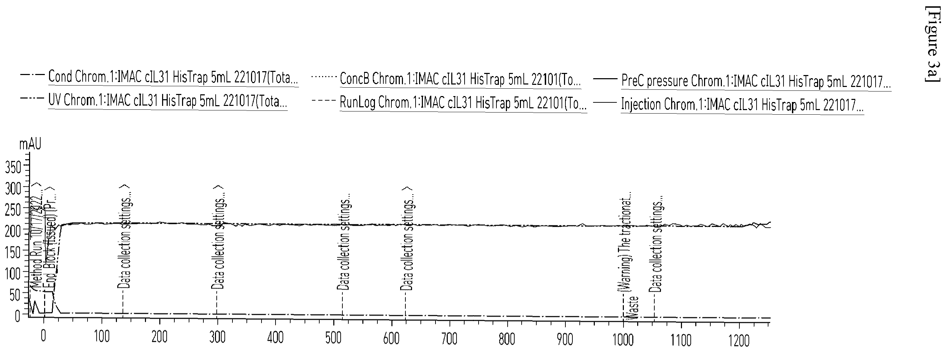

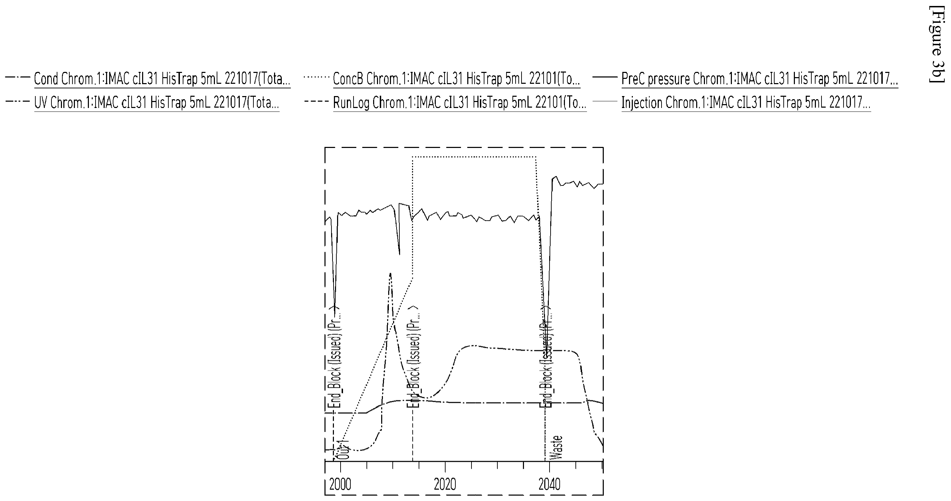

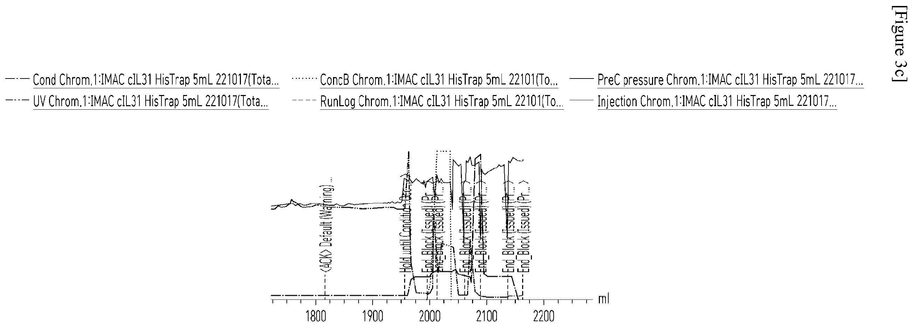

According to the method of one embodiment, the eluted protein was dialyzed against 20 mM phosphate buffer at pH 7.0 to exchange the buffer. The concentration was determined using an absorbance coefficient of 0.766 and was subsequently used for immunization, screening, and other applications (FIGS. 3a-3c).

The quantification formula used is as follows:

$$\text{cIL-13 concentration (mg/mL)} = (A_{280} \times \text{dilution factor}) / 0.766$$

*In the figure: blue line = absorbance at 280 nm; green line = gradient; brown line = conductivity (mS/cm).

1. Purity Analysis

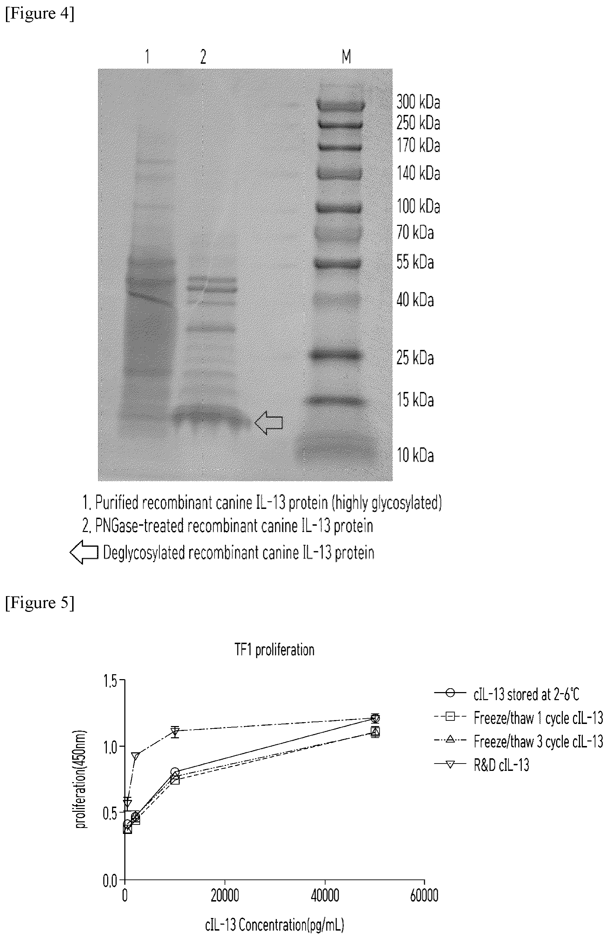

cIL-13 is known to be a heavily glycosylated protein with four N-glycosylation sites. In electrophoresis analysis of the IMAC-purified protein, a distinct major band was not clearly observed. However, upon cleavage of N-linked glycans, the main band is observed to shift to the 10-15 kDa range, which corresponds to the theoretical molecular weight of 13.3 kDa calculated from the amino acid sequence (FIG. 4).

2. Activity Analysis

According to the method of one embodiment, recombinant cIL-13 was added to cultures of the human erythroleukemia cell line TF-1 at various concentrations and incubated for 3 days to evaluate proliferation to verify the activity of cIL-13. For example, cell proliferation was assessed using Ez-Cytox (DoGENBio, #EZ-500), although such examples are not intended to limit the scope of the present disclosure. The produced cIL-13 induced TF-1 cell proliferation in a dose-dependent manner and retained similar activity even after three freeze-thaw cycles (FIG. 5).

**Hybridoma Production**

In one embodiment of the present disclosure, to generate an antibody targeting the recombinant cIL-13 protein, the his-tagged cIL-13 was mixed with ODN 1585-TLR9 ligand, to prepare an immunogen.

According to this method, six-week-old female BALB/c mice were immunized via intraperitoneal injection at intervals of 4 weeks, 3 weeks, and 3 weeks, for a total of three immunizations.

According to one embodiment of this disclosure, one week after the third immunization, blood was collected from the retro-orbital sinus/plexus and plasma was obtained. Immune responses were evaluated by conventional ELISA, which confirmed strong reactivity to the recombinant cIL-13 antigen in all five mice. To proceed with hybridoma development, an additional immunization was administered with the recombinant antigen. Three days later, spleens were harvested from the mice, and splenocyte suspensions were prepared.

[Table 3]

| Preparation of additional Immunogen | |
|---|---|
| Substance | Amount |
| Recombinant His-tagged cIL-13 | 50 $\mu$g/mouse |
| ODN 1585 - TLR9 ligand | 25 $\mu$g/mouse |
| Rat anti-mouse CD40 mAb (1C10) | 30 $\mu$g/mouse |

In one embodiment of the present disclosure, cell fusion was induced using the Köhler-Milstein method. Specifically, $1\times10^8$ splenocytes and $1\times10^7$ myeloma cells were fused in Dulbecco's Modified Eagle Medium (DMEM) using 50% polyethylene glycol 400. The myeloma cell line used may be X63-Ag8.653 (ATCC, PTA-8431), a murine myeloma line resistant to 8-azaguanine (Sigma-Aldrich, #SML2963); however, this example is not intended to limit the scope of the present disclosure.

Following fusion, the cells were washed and resuspended in DMEM supplemented with 20% fetal bovine serum (FBS), 100 $\mu$M hypoxanthine, 0.44 $\mu$M aminopterin, and 16 $\mu$M thymidine (HAT medium). The cell suspension was then plated into 96-well plates and incubated for two weeks in a 37 °C incubator with 5% $CO_2$.

Subsequently, hybridoma pools in which colony formation was observed were screened for antigen reactivity and TF-1 cell proliferation inhibitory activity, followed by the first round of limiting dilution. Afterward, both antigen reactivity and TF-1 proliferation inhibitory activity were re-evaluated, and STAT6 phosphorylation inhibitory activity was assessed, leading to a second round of limiting dilution.

Finally, TF-1 proliferation inhibitory activity was re-evaluated, and five monoclonal purified antibodies were successfully isolated.

**Evaluation of Monoclonal Antibodies**

1. Purity Assessment

In one embodiment of the present disclosure, the physicochemical and biological activities of five subclones of anti-canine IL-13 monoclonal antibodies were comparatively evaluated.

First, the five purified antibodies were analyzed for purity by injecting them into an SE-HPLC column. Each antibody molecule, consisting of two heavy chains and two light chains, was eluted at a retention time of approximately 15.6 minutes and exhibited a high purity of over 96%.

[Table 4]

| Purity Analysis of Monoclonal Antibodies | | | | |
|---|---|---|---|---|
| Clone Name | Peak Identification | Retention Time | Area% | Remarks |
| 32A10-#4 | HMW_1 | 10.76 | 0.22 | |
| | HMW-2 | 13.509 | 3.5 | |
| | Monomer | 15.625 | 96.28 | Main peak |
| 32A10-#17 | HMW_1 | 10.769 | 0.25 | |
| | HMW-2 | 13.508 | 2.8 | |
| | Monomer | 15.618 | 96.95 | Main peak |
| 32A10-#20 | HMW_1 | 10.762 | 0.25 | |
| | HMW-2 | 13.500 | 3.08 | |
| | Monomer | 15.612 | 96.67 | Main Peak |

(continued)

| Purity Analysis of Monoclonal Antibodies | | | | |
|---|---|---|---|---|
| Clone Name | Peak Identification | Retention Time | Area% | Remarks |
| B clone | HMW_1 | 10.772 | 0.39 | |
| | HMW-2 | 13.511 | 3.38 | |
| | Monomer | 15.618 | 96.24 | Main Peak |
| C clone | HMW_1 | 10.777 | 0 | |
| | HMW-2 | 13.673 | 1.99 | |
| | Monomer | 15.660 | 98.01 | Main Peak |

2. Evaluation of Antigen Reactivity

To evaluate the antigen-binding reactivity of the purified antibodies, a cIL-13-coated conventional ELISA assay was performed. Among the clones tested, clone 32A10-#20 exhibited the highest reactivity, with an $EC_{50}$ value of 463 pM.

[Table 5]

| Antigen Reactivity of Mouse Monoclonal Antibodies | | | |
|---|---|---|---|
| Clone Name | EC50 (ng/mL) | EC50(pM) | Remarks |
| 32A10-#4 | 95.59 | 620 | |
| 32A10-#17 | 597.39 | 3,974 | |
| 32A10-#20 | 69.45 | 463 | Final clone |
| B | 92.99 | 620 | |
| C | 79.92 | 533 | |

3. Evaluation of TF-1 Cell Proliferation Inhibitory Activity

Canine IL-13 and human IL-13 share 61.8% similarity in amino acid sequence, and both have been reported to interact with the human IL-13 receptor (Journal of Interferon and Cytokine Research, 20:779-785, 2000). Therefore, to verify the biological activity of the 32A10 monoclonal antibody, its ability to inhibit cell proliferation induced by canine IL-13 was evaluated using the TF-1 cell line.

The inventors of the present invention cultured TF-1 cells in RPMI medium containing 10% fetal bovine serum (FBS) and GM-CSF. On Day 1, the cells were washed to remove GM-CSF. TF-1 cells were then seeded into flat-bottom 96-well microplates at a density of $2 \times 10^4$ cells/50 $\mu$L/well. Recombinant cIL-13 protein in the medium was then added at a concentration of 50 ng/mL, and the antibodies were serially diluted 3-fold starting from 50 $\mu$g/mL and pre-incubated for 1 hour. Then, 100 $\mu$L/well of the IL-13/antibody mixture was added to the TF-1 cells and incubated for an additional 72 hours. Cell proliferation was subsequently evaluated. The 32A10 monoclonal antibody inhibited TF-1 proliferation in a dose-dependent manner, with an $IC_{50}$ in the range of 2.2-3.3 nM.

[Table 6]

| TF-1 Cell Proliferation Inhibitory Activity of Mouse Monoclonal Antibodies | | |
|---|---|---|
| Item | IC50 | |
| Unit | (ng/mL) | nM |
| Test 1 | 493.947 | 3.293 |
| Test 2 | 492.456 | 3.283 |
| Test 3 | 324.939 | 2.166 |
| Average | 437.115 | 2.914 |

4. Binding site

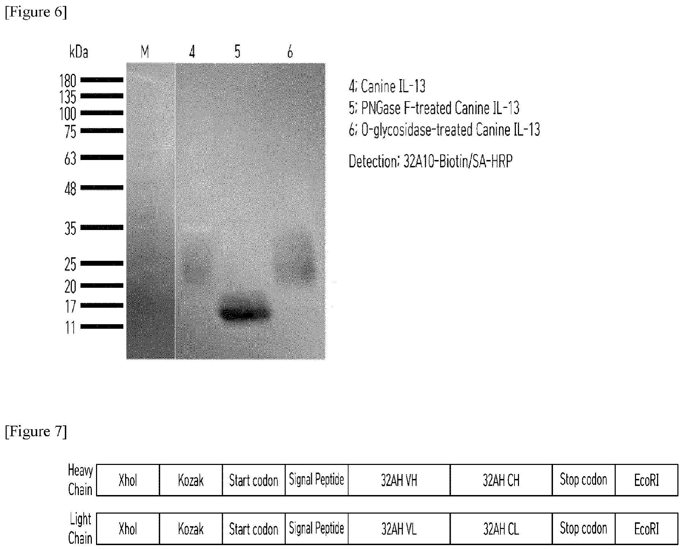

To identify the binding site of the 32A10 antibody, the inventors of the present invention treated recombinant canine IL-13 (cIL-13) protein with PNGase F or O-glycosidase to remove N-linked or O-linked glycans, respectively, and analyzed the binding of the 32A10 monoclonal antibody via Western blotting. The major band of the N-glycan-removed sample appeared at approximately 13 kDa, which corresponds to the theoretical molecular weight of cIL-13, whereas the major bands of the untreated samples and O-glycan-removed samples were observed in the range of 20-35 kDa. The 32A10 antibody reacted regardless of the presence of glycan modifications, indicating that the 32A10 monoclonal antibody recognizes and binds to a peptidic region of canine IL-13 independently of glycans (Figure 6).

**32A10 Gene Sequence Determination**

The antigen-binding specificity and biological activity of a monoclonal antibody are determined by the variable regions, including the complementarity-determining regions (CDRs). To identify the amino acid sequence of the rodent monoclonal antibody 32A10, the inventors of the present invention cloned and obtained the gene sequence from the established 32A10-20 hybridoma.

First, total RNA was isolated from the 32A10 hybridoma. For total RNA isolation, reagents such as the RNeasy Plus Mini Kit (Qiagen) may be used; however, such examples are not intended to limit the scope of the present disclosure. Subsequently, RT-PCR was performed using primers specific to the IgG constant region to obtain the DNA template. PCR amplification was then carried out using the obtained DNA template and variable region-specific primers, and the amplified variable region vectors were obtained via TA cloning. For this process, the Progen Mouse IgG library primer set and TAKARA Mighty TA Cloning Kit may be used; however, these examples are not intended to limit the scope of the present disclosure. Finally, sequencing of three heavy chain and three light chain was commissioned to determine the variable region sequences.

The sequencing results revealed that all CDR sequences were identical regardless of the primer sets used, while two distinct sequences were observed for the framework region 1.

[Table 7]

| Variable Region Sequences of 32A10 | | |
|---|---|---|
| Chain Type | Primer type | Sequence |
| Heavy Chain | C type | QVQLKQSGPELVKPGASVKVSCKTS<u>GYSFTDYP</u>IYWVRQSHGES LEWIGY<u>IDPYNGGT</u>TYNQNFKDKATLTVDKSSSTAFMHLYSLTS EDSAVYFC<u>ATAVVATDVMEN</u>WGQGTSVTVSS |
| | E, F type | EVQLQQSGPELVKPGASVKVSCKTS<u>GYSFTDYP</u>IYWVRQSHESL EWIGY<u>IDPYNGGT</u>TYNQNFKDKATLTVDKSSSTAFMHLNSLTSE DSAVYFC<u>ATAVVATDVMEN</u>WGQGTSVTVSS |

(continued)

| Variable Region Sequences of 32A10 | | |
|---|---|---|
| Chain Type | Primer type | Sequence |
| Light Chain | N type | RRPVNSSSVPGDPIGYSWCSITCKAS<u>QNVRNA</u>VAWFQQKPGQSP KALIY<u>LAS</u>NRYTGVPDHFTGSGSGTDFTLTISNVQSEDLADYFC<u>L QHWKYPLT</u>FGAGTKLPLL |
| | O, Q type | DIVLTQSQKFMSTSVGDRVSITCKAS<u>QNVRNA</u>VAWFQQKPGQS PKALIY<u>LAS</u>NRYTGVPDHFTGSGSGTDFTLTISNVQSEDLADYFC <u>LQHWNYPLT</u>FGAGTKLELK |
| * CDR sequences are underlined and arranged in the order of CDR1, CDR2, and CDR3. | | |

## Cloning of 32A10 Chimeric Antibody

The variable region gene sequences identified from the 32A10 hybridoma were codon-optimized for expression in CHO cells. To transfer the genes into expression vectors, an XhoI restriction enzyme site was added at the 5' end along with a Kozak sequence to promote efficient translation, and a signal peptide derived from human albumin was included to enable secretion of the expressed protein into the extracellular space. At the 3' end, a stop codonto terminate expression and an EcoRI restriction site were added. In one embodiment of the present invention, a canine IgG B-type was used for the heavy chain constant region; however, this example is not intended to limit the scope of the invention. Any of the canine IgG A, B, C, or D types may be used to construct the constant region of the chimeric antibody heavy chain. Similarly, in one embodiment of the invention, a canine kappa light chain constant region was used, but this too is not intended to limit the scope of the invention. Either canine kappa or lambda chains may be used to construct the constant region of the chimeric antibody light chain (Figure 7).

[Table 8]

| Optimized Gene Sequences of the 32A10 Antibody | |
|---|---|
| Type | 32A10-VHC-B type |
| Heavy Chain | TGAAGGGCAAGCAGTTCACCTGCAAGGTGAACAACAAGGCCCT GCCTAGCCCTATCGAGAGAACCATCAGCAAGGCTAGAGGCCAA GCCCATCAGCCTAGCGTGTACGTGCTGCCTCCTAGCAGAGAGGA |

| Optimized Gene Sequences of the 32A10 Antibody | |
|---|---|
| Type | 32A10-VHC-B type |
| | GCTGAGCAAGAACACCGTGAGCCTGACCTGCCTGATCAAGGACTTCTTCCCTCCTGACATCGACGTGGAGTGGCAGAGCAACGGACAGCAAGAGCCTGAGAGCAAGTACAGAACCACCCCTCCTCAGCTGGACGAGGACGGCAGCTACTTCCTGTACAGCAAGCTGAGCGTCGACAAGAGCAGATGGCAGAGAGGCGACACCTTCATCTGCGCCGTGATGCACGAGGCCCTGCACAACCACTACACCCAAGAGAGCCTGAGCCACAGCCCTGGCAAGTGAGAATTC |
| Type | 32A10-VHF-B type |
| Heavy Chain | AGGGCAAGCAGTTCACCTGCAAGGTGAACAACAAGGCCCTGCCTAGCCCTATCGAGAGAACCATCAGCAAGGCTAGAGGCCAAGCCCATCAGCCTAGCGTGTACGTGCTGCCTCCTAGCAGAGAGGAGCTGAGCAAGAACACCGTGAGCCTGACCTGCCTGATCAAGGACTTCTTCCCTCCTGACATCGACGTGGAGTGGCAGAGCAACGGACAGCAAGAGCCTGAGAGCAAGTACAGAACCACCCCTCCTCAGCTGGACGAGGACGGCAGCTACTTCCTGTACAGCAAGCTGAGCGTCGACAAGAGCAGATGGCAGAGAGGCGACACCTTCATCTGCGCCGTGATGCACGAGGCCCTGCACAACCACTACACCCAAGAGAGCCTGAGCCACAGCCCTGGCAAGTGAGAATTC |
| Type | 32A10-VLN-Kappa |
| Light Chain | CTCGAGGCCACCATGAAGTGGGTGACCTTCATCAGCCTGCTGTTCCTGTTCAGCAGCGCCTACAGCAGAAGACCTGTGAACAGCAGCAGCGTGCCTGGCGACCCTATCGGCTACAGCTGGTGCAGCATCACCTGCAAGGCTTCTCAGAACGTGAGAAACGCCGTGGCCTGGTTTCAGCAGAAGCCTGGACAGAGCCCTAAGGCCCTGATCTACCTGGCT |

(continued)

| Optimized Gene Sequences of the 32A10 Antibody | |
|---|---|
| Type | 32A10-VHC-B type |
| | AGCAACAGATACACCGGCGTGCCTGACCACTTCACCGGCAGCG GCAGCGGCACCGACTTCACGCTGACCATCAGCAACGTGCAGAG CGAGGACCTGGCCGACTACTTCTGCCTGCAGCACTGGAAGTACC CTCTGACCTTCGGCGCCGGCACCAAGCTGCCTCTGCTGAGAAAC GACGCTCAGCCTGCCGTGTACCTGTTTCAGCCTAGCCCTGATCA GCTGCACACCGGCAGCGCTAGCGTGGTGTGCCTGCTGAACAGCT TCTACCCTAAGGACATCAACGTGAAGTGGAAGGTGGACGGCGT GATCCAAGACACCGGCATCCAAGAGAGCGTGACCGAGCAAGAC AAGGACAGCACCTACAGCCTGAGCAGCACCCTAACCATGAGCA GCACCGAGTACCTGAGCCACGAGCTGTACAGCTGCGAGATCACC CACAAGAGCCTGCCTAGCACCCTGATCAAGAGCTTTCAGAGAAG CGAGTGCTGAGAATTC |
| Type | 32A10-VLO-Kappa |
| Light Chain | CTCGAGGCCACCATGAAGTGGGTGACCTTCATCAGCCTGCTGTT CCTGTTCAGCAGCGCCTACAGCGACATCGTGCTGACACAGTCTC AGAAGTTCATGAGCACAAGCGTGGGCGACAGAGTGAGCATCAC CTGCAAGGCTTCTCAGAACGTGAGAAACGCCGTGGCCTGGTTTC AGCAGAAGCCTGGACAGAGCCCTAAGGCCCTGATCTACCTGGCT AGCAACAGATACACCGGCGTGCCTGACCACTTCACCGGCAGCG GCAGCGGCACCGACTTCACGCTGACCATCAGCAACGTGCAGAG CGAGGACCTGGCCGACTACTTCTGCCTGCAGCACTGGAACTACC CTCTGACCTTCGGCGCCGGCACCAAGCTGGAGCTGAAGAGAAA CGACGCTCAGCCTGCCGTGTACCTGTTTCAGCCTAGCCCTGATC AGCTGCACACCGGCAGCGCTAGCGTGGTGTGCCTGCTGAACAGC |

(continued)

| Optimized Gene Sequences of the 32A10 Antibody | |
|---|---|
| Type | 32A10-VHC-B type |
| | TTCTACCCTAAGGACATCAACGTGAAGTGGAAGGTGGACGGCGT GATCCAAGACACCGGCATCCAAGAGAGCGTGACCGAGCAAGAC AAGGACAGCACCTACAGCCTGAGCAGCACCCTAACCATGAGCA GCACCGAGTACCTGAGCCACGAGCTGTACAGCTGCGAGATCACC CACAAGAGCCTGCCTAGCACCCTGATCAAGAGCTTTCAGAGAAG CGAGTGCTGAGAATTC |

The four chimeric gene constructs described in Table 8 were individually inserted into a pcDNA3.4 expression vector in which the multiple cloning site had been modified with XhoI and EcoRI, thereby generating the expression vectors.

To construct the expression vectors encoding the chimeric genes described in Table 8, a method of the present disclosure first involved double digestion of the four DNA constructs using XhoI and EcoRI, followed by gel electrophoresis and gel extraction to purify the insert DNAs. The pcDNA3.4 vector was also double-digested with XhoI and EcoRI, subjected to electrophoresis, and gel-extracted. Subsequently, the inserts (two heavy chain variants and two light chain variants) were ligated into the pcDNA3.4 vector using T4 DNA ligase, and the resulting ligation mixtures were transformed into E. coli DH5α cells. As a result, the following four recombinant plasmid strains were generated: pcDNA3.4-VHC-B, pcDNA3.4-VHF-B, pcDNA3.4-VLN-kappa, and pcDNA3.4-VLO-kappa. To confirm successful insertion of target genes into the vectors, the plasmids were subjected once again to double digestion with XhoI and EcoRI, followed by electrophoresis. The sequences of the variable regions of the heavy and light chains were then verified via DNA sequencing.

**Production of 32A10 Mouse/Canine Chimeric Antibodies**

In one embodiment of the present disclosure, two heavy chain types (C-type and F-type) and two light chain types (N-type and O-type) were combinatorially transfected into CHO cells, which were cultured until cell viability below 70%. The culture fluid was then harvested and applied to a chromatography column to purify the chimeric antibodies. Although a MabSelect SuRe column (Cytiva, #29049104) may be used for this purpose, such an example is not intended to limit the scope of the present disclosure. Among the four combinations tested, antibody production was observed only in the two combinations that included the O-type light chain, indicating that the N-type light chain gene likely contains an incorrect sequence that does not support antibody expression. The purified antibodies were quantified by measuring absorbance at 280 nm (OD280), and their purity and activity were subsequently evaluated.

[Table 9]

| Transient Culture of Mouse/Canine 32A10 Chimeric Monoclonal Antibodies | | | | | |
|---|---|---|---|---|---|
| Name | Variable Heavy Chain Region | Constant Heavy Chain Region | Variable Light chain Region | Constant Light Chain Region | Culture volume |
| Chimeric 32A10 CN | C-type | subtype B | N- type | Kappa | 50 mL |
| Chimeric 32A10 CO | C- type | subtype B | O- type | Kappa | 50 mL |
| Chimeric 32A10 FN | F- type | subtype B | N- type | Kappa | 50 mL |
| Chimeric 32A10 FO | F- type | subtype B | O- type | Kappa | 50 mL |

1. Purity Assessment

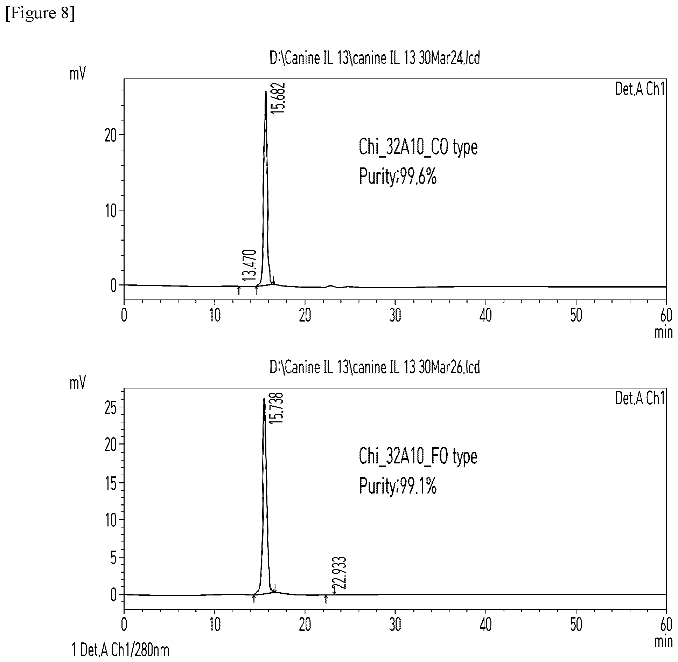

The inventors of the present invention evaluated the purity of two purified chimeric antibodies by injecting them into a SE-HPLC column. A TSKgel G3000SWXL column (TOSOH) may be used for this purpose; however, such an example is not intended to limit the scope of the present disclosure. The antibody molecule consisting of two heavy chains and two light chains was eluted at approximately 15.6 minutes of retention time, and both antibodies exhibited high purity exceeding 99% (Figure 8).

2. Antigen Reactivity Assessment ($EC_{50}$ Quantification)

In one embodiment of the present disclosure, recombinant canine IL-13 (cIL-13) protein with a histidine tag was dispensed onto nickel-coated plates (Thermo Fisher, #15442) at 100 ng/100 μL per well and incubated at room temperature for 1 hour. The plates were then washed three times with $1\times$ PBST, and serial dilutions of the chimeric antibodies in $1\times$ PBS were added and incubated at room temperature for 1 hour to allow antigen-antibody binding. After three washes with $1\times$ PBST, an HRP-conjugated secondary antibody that cross-reacts with canine antibodies was diluted 1:100 in casein buffer and added at 100 μL per well, followed by incubation at 37°C for 30 minutes. The HRP-conjugated secondary antibody used may be Jackson ImmunoResearch, #115-036-071, and the casein buffer may be Thermo Fisher Scientific, #37528; however, these examples are not intended to limit the scope of the present disclosure. After another three washes with $1\times$ PBST, TMB was added to initiate color development, and the reaction was terminated by the addition of an equal volume of 1.0 N sulfuric acid. The $EC_{50}$ values of the two chimeric antibodies were found to be similar to that of the parental mouse 32A10 antibody.

[Table 10]

| Binding Reactivity to Canine Interleukin-13 | | |
|---|---|---|
| Antibody | EC50 (ng/mL) | EC50 (nM) |
| Parental antibody (Mouse 32A10) | 217.9 | 1.45 nM |
| Chimeric antibody (CO type) | 162.4 | 1.08 |
| Chimeric antibody (FO type) | 147.3 | 0.98 nM |

3. Inhibition Activity of TF-1 Cell Proliferation

The ability of the chimeric antibody to inhibit proliferation of the TF-1 cell line was evaluated. In one embodiment of the present disclosure, TF-1 cells were dispensed the day before the assay (Day -1) at a density of $2\times10^4$ cells/50 μL/well in media lacking GM-CSF and incubated overnight. On the day of the assay, the parental antibody or the chimeric antibody FO was serially diluted 3-fold to prepare 10 test samples ranging from 50,000 to 2.54 ng/mL. Recombinant canine IL-13 (cIL-13) antigen solution was prepared at 300 ng/mL and mixed in equal volume with the diluted antibody sample solutions to induce antigen-antibody neutralization reactions (incubated at 37°C for 1 hour). Then, 100 μL of each antigen-antibody mixture fluid was added to the TF-1 cell culture, followed by 72 hours of incubation.
After incubation, the proliferation of the TF-1 cells was measured. The chimeric antibody FO exhibited TF-1 growth inhibition comparable to that of the parental antibody.

[Table 11]

| Inhibitory Activity on TF-1 Cell Proliferation | |
|---|---|
| Antibody | IC50 (pM) |
| Parental antibody (Mouse 32A10) | 3,969 pM |
| Chimeric antibody (FO type) | 3,278 pM |

4. Inhibition Activity of STAT-6 Phosphorylation

To evaluate the ability of the chimeric antibody to block downstream signaling of cIL-13, its inhibitory effect on STAT-6 phosphorylation was assessed.
In one embodiment of the present disclosure, TF-1 cells were suspended and washed in GM-CSF-free medium on the day before the assay (Day -1), then plated at $4\times10^5$ cells/50 μL/well and incubated for 24 hours at 37°C in a $CO_2$

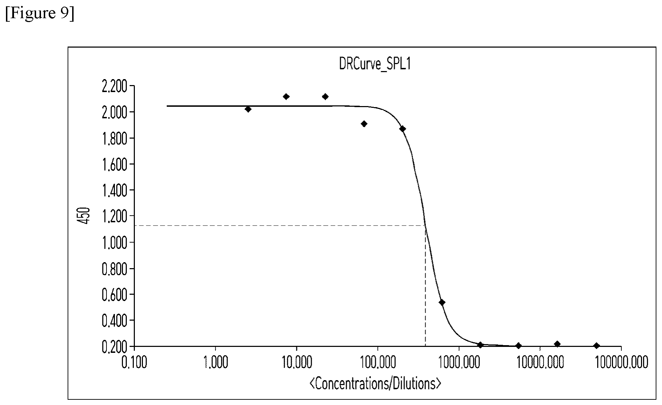

incubator. In one embodiment of the present disclosure, the chimeric antibody was serially diluted in medium containing 100 ng/mL of IL-13 to prepare 9 samples ranging from 50,000 to 7.62 ng/mL. Each 200 $\mu$L mixture was incubated at 37°C in a $CO_2$ incubator for 1 hour to allow antigen-antibody interaction and then added to the TF-1 cells at 50 $\mu$L/well. After a 5-minute stimulation at 37°C with 5% $CO_2$ to induce intracellular signaling, the TF-1 cells were harvested and washed with 1× PBS. Three wells were prepared for identical condition, and to ensure assay sensitivity, the contents of the three wells were pooled and analyzed as a single sample. Intracellular phosphorylated STAT-6 was quantified using the PathScan® Phospho-STAT6 (Tyr641) Sandwich ELISA Kit (Cell Signaling Technology, #7275C), following the manufacturer's instructions; however, this example is not intended to limit the scope of the present disclosure. The chimeric antibody FO effectively inhibited IL-13-induced STAT-6 phosphorylation in a concentration-dependent manner, with an $IC_{50}$ of 2,418 pM. (Figure 9)

[Table 12]

| Amino Acid Sequences of the 32A10 Variable Regions | |
|---|---|
| Type | Amino Acid Sequences of the 32A10 Variable Regions |
| VH | EVQLQQSGPELVKPGASVKVSCKTS<u>GYSFTDYP</u>IYWVRQSHGESL EWIGY<u>IDPYNGGT</u>TYNQNFKDKATLTVDKSSSTAFMHLNSLTSED SAVYFC<u>ATAVVATDVME</u>NWGQGTSVTVSS |
| VL | DIVLTQSQKFMSTSVGDRVSITCKAS<u>QNVRNA</u>VAWFQQKPGQSP |
| | KALIY<u>LAS</u>NRYTGVPDHFTGSGSGTDFTLTISNVQSEDLADYFC<u>LQ</u> <u>HWNYPLT</u>FGAGTKLELK |

**Caninized Antibody Design**

To develop the recombinant antibody as a therapeutic for canine atopic dermatitis, further caninization of the established chimeric antibody was deemed necessary. In the present disclosure, "caninization" refers to the process of partially replacing the murine-derived variable region of a chimeric antibody with the canine variable region to reduce adverse effects caused by immunogenicity when the antibody is administered to dogs. To reduce immunogenicity while maintaining antigen-binding ability, one embodiment of the present disclosure employed CDR grafting technology to transplant the murine complementarity-determining regions (CDRs) of the chimeric antibody into canine germline-derived framework regions. In this context, the antibody providing the CDRs is referred to as the donor, and the antibody receiving the CDRs within its structural framework is referred to as the recipient.

In one embodiment of the present disclosure, homology model building was performed to carry out CDR grafting. Homology modeling refers to constructing a three-dimensional model of the antibody using the established sequences of the chimeric antibody, and is considered helpful particularly for predicting the structure of the CDRs or for assessing structural compatibility between the framework and CDR regions. Homology modeling can also assist in deciding whether to retain donor residues or replace them with recipient residues, thus laying the foundation for the overall caninization process.

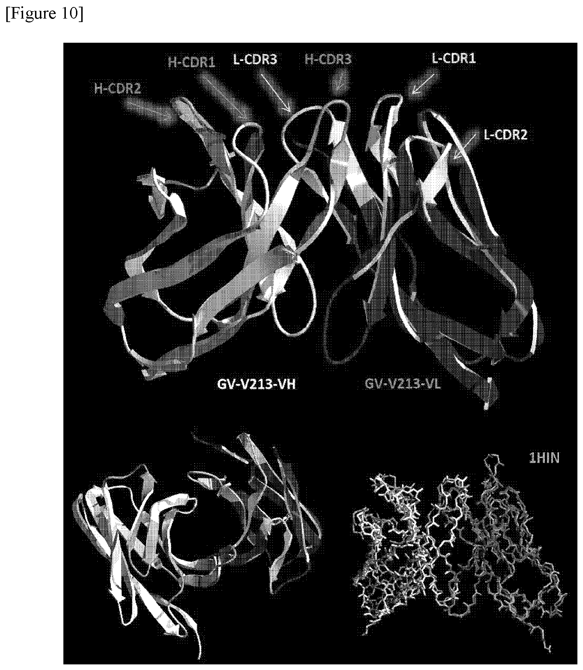

In one embodiment of the present disclosure, to build the homology model, protein structures with high sequence homology to the variable region sequences of the chimeric antibody light and heavy chains were searched using the Protein Data Bank (PDB). The structure of PDB code 5IKC was selected as the framework template for the light chain variable region, and the structure of 5YFI was selected as the framework template for the heavy chain variable region. A deep learning-based analytical system was used to analyze the CDR loop structures. Although the deep learning-based CDR loop analysis system Ablooper may be used, it is not limited thereto. In one embodiment of the present disclosure, the canonical structures(Chothia standard conformations) corresponding to the three CDRs of the light and heavy chain regions were identified, and separate template structures were selected for each CDR. Next, appropriate three-dimensional structural models of the heavy and light chain variable region sequences were identified through a protein-binding prediction program. According to one embodiment of the present disclosure, the three-dimensional structure suitable for the variable regions of the chimeric antibody was that of the protein assigned PDB code 1HIN in the Protein Data Bank, which has a relative packing angle of -46.9°. Although the prediction programs PAPS and ABangle may be used, they are not limited thereto. In one embodiment of the present disclosure, the final model was assembled by aligning the backbone coordinates of the chimeric antibody's anchor residues with the structure of 1HIN, and energy minimization was performed through molecular dynamics simulation. A schematic of the final model is shown in Figure 10.

When designing the caninized antibody sequence, careful attention must be paid to whether murine residues should be retained or replaced with canine residues to prevent loss of affinity or antibody stability due to structural incompatibility between the murine CDRs and canine frameworks. This consideration is especially important at Vernier zones. Vernier zones are located within or adjacent to the CDRs structurally and are considered to influence CDR conformation and fine-tuning of antigen recognition. Replacement with canine residues aims to increase sequence identity to canine genes without compromising antibody affinity. Additionally, in designing antibody candidates, a comprehensive sequence optimization strategy should be considered to improve the physicochemical properties, manufacturability, and pharmacological properties of the antibody. Given that antibodies are proteins and thus physically vulnerable, it is desirable to design them to maintain chemical and structural integrity during manufacturing, distribution, and storage. Therefore, the possibility of various post-translational modifications (PTMs) was also considered. The possibilities of methionine oxidation, N- and O-glycosylation, asparagine deamidation, aspartate (D) isomerization, N-terminal glutamine cyclization, cysteine bonding, lysine side-chain glycation, and tryptophan oxidation were each examined. Certain residues were caninized or retained as murine in order to avoid such PTMs.

In one embodiment of the present disclosure, to select recipient frameworks for grafting the CDRs of the chimeric antibody, similar sequences to the heavy and light chain variable regions were searched using the canine gene database and the Canine Genome Project. Selection was made based on overall framework sequence identity, conformity and compatibility of the contact points between heavy and light variable regions, and CDR canonical structures.

According to the analysis, for the variable heavy chain region, the canine germline genes IGHV1-30*01 and IGHV3-23*01 were selected as suitable templates. For the variable light chain region, IGK3-18*01 and IGKV2-10*01 were selected as suitable templates. For each selected template, three versions of caninized sequences were designed.

[Table 13]

Caninized Antibody Sequences

| | | | Sequence | Sequ ence No. |
|---|---|---|---|---|
| V H | IGH V1- | V 1 | EVQLVQSGAEVVKPGASVKVSCKTSGYSFTDYPIYWVRQAPGAGLE WIGYIDPYNGGTTYNQNFKDRVTLTADKSTSTAYMELSSLRAGDIA | 17 |

| Caninized Antibody Sequences | | | | |
|---|---|---|---|---|
| | | | Sequence | Sequ ence No. |
| | 30*0 1 | | VYYCATAVVATDVMENWGQGTLVTVSS | |
| | | V 2 | EVQLVQSGAEVKKPGASVKVSCKTSGYTFIDYPMYWVRQAPGAGL EWMGYIDPYNGATSYNQNFKGRVTLTADKSTSTAYMELSSLRAGDI AVYYCATAVVATDVMENWGQGTLVTVSS | 18 |
| | | V 3 | EVQLVQSGAEVKKPGASVKVSCKTSGYTFIDYPMYWVRQAPGAGL EWMGYIDPYNGATSYNQKFQGRVTLTADTSTSTAYMELSSLRAGDI AVYYCARAVVATDVMENWGQGTLVTVSS | 19 |
| | IGH V3-23*0 1 | V 1 | EVQLVQSGGELVKPGGSLRLSCKTSGYSFTSYPIYWVRQAPGKGLQ WVGYIDPYNGGTTYNQNFKDRATLSVDKASSTAYMQLSSLRAEDT AVYFCATAVVATDVMENWGQGTLVTVSS | 20 |
| | | V 2 | EVQLVQSGGELEKPGGSLRLSCVASGFTFSDYPMYWVRQAPGKGLQ WVSYIDPYNSGTTYNQNVKGRFTISVDKAKSTLYLQMSSLRAEDTA VYSCATAVVATDVMENWGQGTLVTVSS | 21 |
| | | V 3 | EVQLVQSGGELEKPGGSLRLSCVASGFTFSSYGMYWVRQAPGKGLQ WVSYIDYYNSGTTYNQAVKGRFTISRDKAKSTLYLQMSSLRAEDTA VYSCAKAVVATDVMENWGQGTLVTVSS | 22 |

EP 4 671 274 A1

| Caninized Antibody Sequences | | | | |
|---|---|---|---|---|
| | | | Sequence | Sequ ence No. |
| V L | IGK V3-18*0 1 | V 1 | EIVLTQSPASLSLSQEERVTITCKASQNVRNAVAWFQQKPGQAPKAL IYLASNRYTGVPSRFSGSGSGTDFTLTISSLEPEDVADYFCLQHWNYP LTFGAGTKVELK | 23 |
| | | V 2 | EIVMTQSPASLSLSQEERVTITCRASQNVRNALAWYQQKPGQAPKLL IYLTSNRYTGVPSRFSGSGSGTDFTFTISSLEPEDVAVYYCLQHWNYP LTFGAGTKVELK | 24 |
| | | V | EIVMTQSPASLSLSQEERVTITCRASQSVRSALAWYQQKPGQAPKLLI | 25 |

EP 4 671 274 A1

(continued)

| Caninized Antibody Sequences | | | | |
|---|---|---|---|---|
| | | | Sequence | Sequ ence No. |
| | | 3 | YGTSNRYTGVPSRFSGSGSGTDFTFTISSLEPEDVAVYYCLQHWSYPL TFGAGTKVELK | |
| | IGK V2-10*0 1 | V 1 | DIVLTQTPLSLSVSPGETASISCKASQNVRNAVAWFRQKPGQSPQALI YLASNRYTGVPDRFSGSGSGTDFTLRISRVEAEDTGDYFCLQHWNYP LTFGAGTKVELK | 26 |
| | | V 2 | DIVMTQTPLSLSVSPGETASISCKASQNLRNALAWFRQKPGQSPQALI YLVSNRYPGVPDRFSGSGSGTDFTLRISRVEAEDTGVYYCLQHWQY PLTFGAGTKVELK | 27 |
| | | V 3 | DIVMTQTPLSLSVSPGETASISCKASQSLRSALAWFRQKPGQSPQRLI YLVSNRYPGVPDRFSGSGSGTDFTLRISRVEAEDTGVYYCLQHWQFP LTFGAGTKVELK | 28 |

Accordingly, in one embodiment of the present disclosure, antibodies to be produced were selected based on the results of the above-described caninized antibody design, as shown in Table 14.

[Table 14]

| Nomenclature of Designed Caninized Antibodies | | | | | | | | |
|---|---|---|---|---|---|---|---|---|
| Canine derived germline sequence | | | VL | | | | | |
| | | | IGKV3-18*01 | | | IGKV2-10*01 | | |
| | | | V1 | V2 | V3 | V1 | V2 | V3 |
| VH | IGHV1-30*01 | V1 | Ab21 | Ab3 | Ab6 | Ab22 | Ab23 | Ab12 |
| | | V2 | Ab1 | Ab4 | Ab7 | Ab24 | Ab10 | Ab13 |
| | | V3 | Ab2 | Ab5 | Ab8 | Ab9 | Ab11 | Ab14 |
| | IGHV3-23*01 | V1 | - | - | - | - | - | - |
| | | V2 | - | Ab15 | Ab17 | - | - | - |
| | | V3 | - | Ab16 | Ab18 | - | Ab19 | Ab20 |

**Production of Caninized Antibodies**

The inventors of the present disclosure commissioned an external organization to produce the selected caninized antibodies, as listed in Table 14. Among them, antibodies Ab1 through Ab14 and Ab21 through Ab24 were successfully produced, whereas antibodies Ab15 through Ab20 were, unfortunately, not successfully generated. All successfully produced antibodies exhibited a purity greater than 98%, and their endotoxin levels were below 1 EU. The purity and endotoxin levels of antibodies Ab1 through Ab24 are summarized in Table 15.

[Table 15]

| Purity and Endotoxin Levels of Ab1 to Ab24 | | | |
|---|---|---|---|
| | Purity %(SEC-HPLC TEST) | Endotoxin, EU/mg | Production amount (mg) |
| Ab1 | 100% | <1EU/mg | 1.09 |
| Ab2 | 100% | <1EU/mg | 1.16 |
| Ab3 | 100% | <1EU/mg | 0.67 |
| Ab4 | 100% | <1EU/mg | 0.69 |
| Ab5 | 100% | <1EU/mg | 0.50 |
| Ab6 | 100% | <1EU/mg | 1.08 |
| Ab7 | 100% | <1EU/mg | 0.91 |
| Ab8 | 100% | <1EU/mg | 0.71 |
| Ab9 | 98.804% | <1EU/mg | 1.09 |
| Ab10 | 100% | <1EU/mg | 1.36 |
| Ab11 | 100% | <1EU/mg | 0.88 |
| Ab12 | 100% | <1EU/mg | 0.77 |
| Ab13 | 100% | <1EU/mg | 058 |
| Ab14 | 100% | <1EU/mg | 0.54 |
| Ab21 | 99.54% | 0-1EU/mg | 2.43 |
| Ab22 | 100% | 0-1EU/mg | 2.7 |
| Ab23 | 100% | 0-1EU/mg | 1.08 |
| Ab24 | 98.86% | 0-1EU/mg | 2.08 |

(continued)

| Purity and Endotoxin Levels of Ab1 to Ab24 | | | |
|---|---|---|---|
| | Purity %(SEC-HPLC TEST) | Endotoxin, EU/mg | Production amount (mg) |
| Positive Control Ab | 100% | <1EU/mg | 0.48 |

**Evaluation of Caninized Antibodies**

In one embodiment of the present disclosure, to verify the function of the successfully produced caninized antibodies and identify clinical candidate molecules, the antibodies were evaluated for their binding ability to cIL-13, thermal stability, and TF-1 cell proliferation inhibition activity.

1. Antigen Reactivity ($EC_{50}$)

A conventional ELISA method was used to assess the antigen reactivity of the caninized antibodies. The inventors of the present invention coated 96-well nickel plates with His-tagged cIL-13 at a concentration of 1 $\mu$g/mL and incubated the plates at room temperature for 1 hour. Wells were washed three times using 200 $\mu$L of wash buffer per well; the wash buffer consisted of 1$\times$ PBS containing 0.1% Tween-20. In one embodiment of the present disclosure, diluted antibodies were added to the wells at 100 $\mu$L per well and incubated again at room temperature for 1 hour. The dilution buffer used was a 1:1 mixture of 25 mL of 1$\times$ casein solution and 25 mL of 1$\times$ PBS. Next, 50 $\mu$L of HRP-conjugated goat anti-human IgG was diluted in 10 mL of dilution buffer and dispensed at 100 $\mu$L per well, followed by incubation at room temperature for 1 hour. The wells were then washed three times with 200 $\mu$L of wash buffer. TMB substrate (50 $\mu$L per well) was added. The plates were incubated at room temperature for 10 minutes. The reaction was stopped by adding 50 $\mu$L per well of 1 N sulfuric acid as a stop solution, and absorbance was measured at 450 nm using a spectrophotometer. Based on the results, $EC_{50}$ values were calculated as shown in Table 16. Antibodies Ab1 through Ab5 demonstrated superior antigen reactivity compared to the chimeric antibody, while Ab21 through Ab24 showed antigen reactivity comparable to the chimeric antibody.

[Table 16]

| $EC_{50}$ Values of Ab1 to Ab24 | |
|---|---|
| | Ag EC50 (ng/mL) |
| Ab1 | 56.4 |
| Ab2 | 78.16 |
| Ab3 | 45.95 |
| Ab4 | 57.2 |
| Ab5 | 76.48 |
| Ab6 | 246.37 |
| Ab7 | N.D |
| Ab8 | 12350.66 |
| Ab9 | 92.65 |
| Ab10 | 81.95 |
| Ab11 | 110.62 |
| Ab12 | 4386.52 |
| Ab13 | N.D |
| Ab14 | 1775.9 |
| Ab21 | 122.482 |
| Ab22 | 137.368 |
| Ab23 | 107.821 |
| Ab24 | 133.509 |

(continued)

| EC$_{50}$ Values of Ab1 to Ab24 | |
|---|---|
| | Ag EC50 (ng/mL) |
| Chimeric antibody(Average) | 92.84 |

2. Thermal Stability

Under thermal conditions, antibody instability may lead to reduced production yield, loss or degradation of efficacy, noxious immune responses, and patient-related complications. Moreover, antibody function may be limited or lost when exposed to extreme conditions or during long-term storage. Therefore, thermal stability is considered an important indicator that may influence future application. In one embodiment of the present disclosure, each test sample was aliquoted at 200 $\mu$L and stored for 1 hour at either 60°C or 4°C. Subsequently, the inventors of the present invention dispensed 180 $\mu$L of each sample into wells of an opaque 96-well plate. Then, 20 $\mu$L of a 0.2 mg/mL ANS solution was added to each well and incubated in the dark for 30 minutes. The plate was analyzed using a fluorescence reader (Excitation: 375 nm, Emission: 470 nm).
To quantify antibody thermal stability, the following formula was used:

$$\text{Relative Instability (\%)} = [(\text{Thermal stress at } 60°C - \text{Untreated}) / \text{Untreated}] \times 100$$

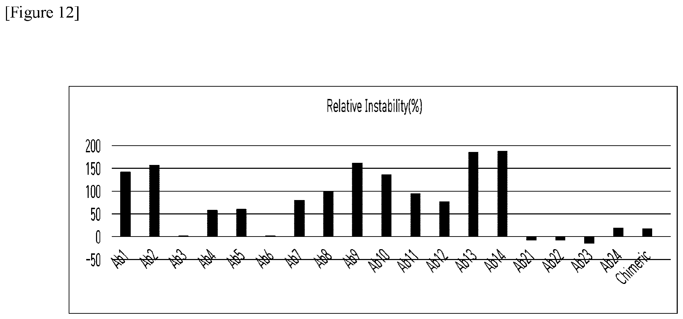

The thermal stability comparison results for each antibody candidate are shown in Figure 12 and Table 17. Antibodies Ab21 through Ab24 exhibited thermal stability that was comparable to or better than that of the chimeric antibody.

[Table 17]

| Relative Instability of Ab1 to Ab24 | |
|---|---|
| Antibody Name | Relative Instability (%) |
| Ab1 | 139.9 |
| Ab2 | 155.4 |
| Ab3 | 1.3 |
| Ab4 | 57.5 |
| Ab5 | 60.8 |
| Ab6 | 1.5 |
| Ab7 | 79.3 |
| Ab8 | 97.7 |
| Ab9 | 160.3 |
| Ab10 | 135.0 |
| Ab11 | 93.2 |
| Ab12 | 75.0 |
| Ab13 | 184.7 |
| Ab14 | 184.9 |
| Ab21 | -7.2 |
| Ab22 | -8.1 |
| Ab23 | -14.5 |
| Ab24 | 17.8 |
| Chimeric antibody | 18.0 |

1. TF-1 Cell Proliferation Inhibition Activity

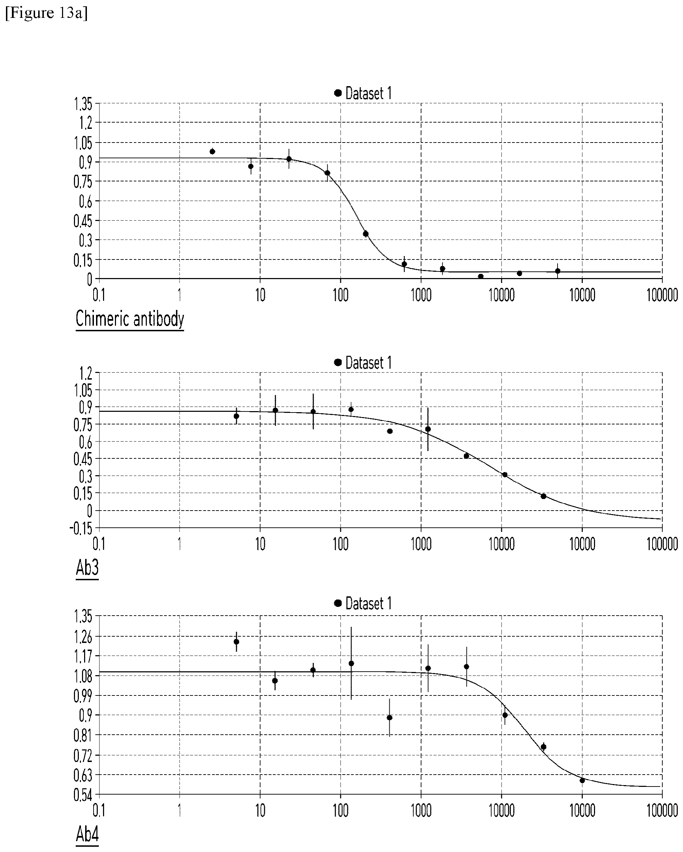

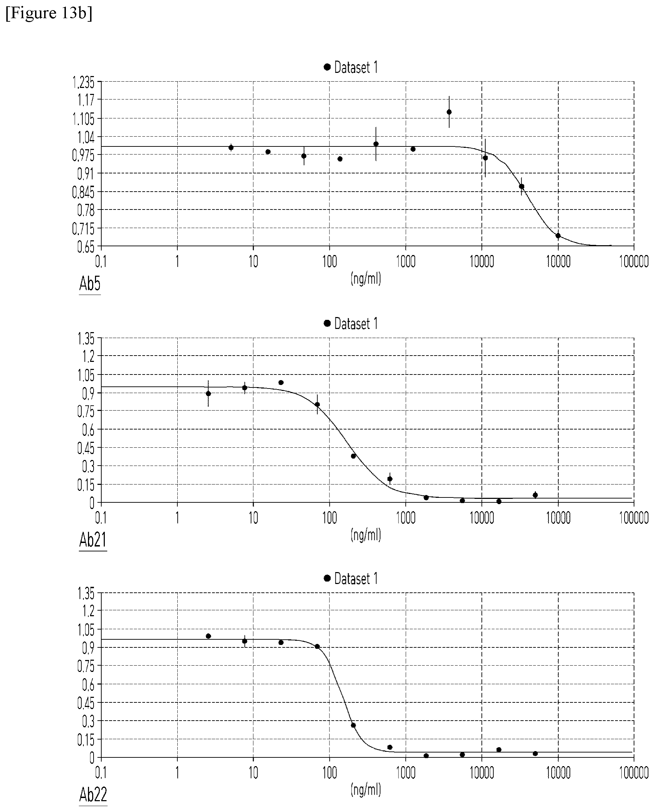

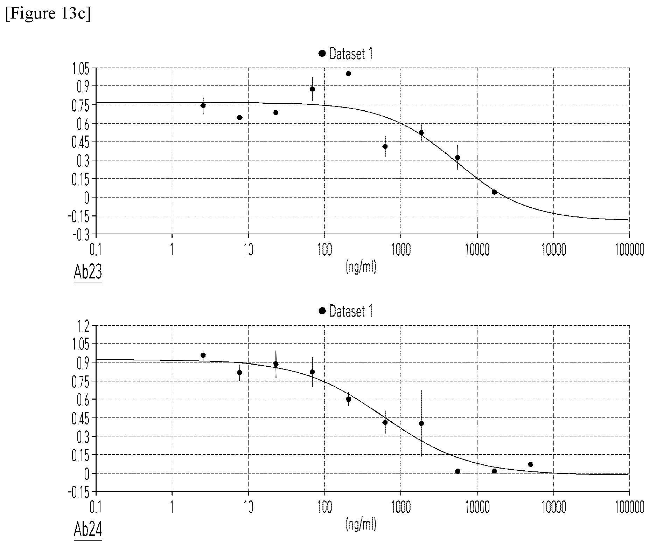

In one embodiment of the present disclosure, the cell proliferation inhibitory effect of the caninized antibodies was evaluated. The inventors of the present invention plated TF-1 cells at $2\times10^4$ cells per 50 μL per well under GM-CSF-free conditions one day prior to the experiment, except in wells B2, B3, and B4. The cells were incubated overnight at 37°C in a 5% $CO_2$ incubator. Then, 90 μL of antibody dilution at a concentration of 300 μg/mL was dispensed into wells B11, C11, D11, E11, F11, and G11 of a transparent 96-well V-bottom plate. Using a multichannel pipette, 30 μL of the antibody solution was transferred sequentially from wells B11, C11, D11, E11, F11, and G11 to wells B10, C10, D10, E10, F10 and G10, followed by repeated pipetting for thorough mixing. This procedure was repeated down to wells B2, C2, D2, E2, F2, and G2, from which 30 μL was discarded to adjust the final volume to 60 μL. In one embodiment of the present disclosure, 60 μL of media was added to wells A2-A7 and H2-H7. Then, 60 μL of cIL-13 solution, prepared at a concentration of 100 ng/mL, was added to each well containing 60 μL of diluted antibody solution and IL-13 only. For wells with only TF-1 cells and only GM-CSF, 120 μL of media was added. The plate was incubated for 1 hour at 37°C in a 5% $CO_2$ incubator. To each well of TF-1 cell plate that had been incubated overnight, 100 μL of the cIL-13/antibody mixture was added to bring the final volume to 150 μL. The plate was then incubated for 72 hours at 37°C in a 5% $CO_2$ incubator. Before the end of the incubation period, a substrate solution for the proliferation inhibition assay was prepared. 20 μL of the substrate was mixed with 30 μL of media per well. After incubation, 50 μL of this substrate solution was added to each well, and the plate was further incubated for 3 hours at 37°C in a 5% $CO_2$ incubator. After equilibrating at room temperature for 15 minutes, absorbance was measured using a spectrophotometer. Based on the measurements, $IC_{50}$ values were calculated as shown below. The TF-1 cell proliferation inhibition activities of the chimeric antibody and antibodies Ab3-Ab5 and Ab21-Ab24 are illustrated in Figures 13a-13c. Upon comparison, Ab21 and Ab22 demonstrated similar inhibitory activity to the chimeric antibody 32A10. However, Ab3-Ab5 and Ab23-Ab24 were found to lack sufficient cell proliferation inhibitory potency.

[Table 18]

| IC$_{50}$ Values of Ab3 to Ab5 and Ab21 to Ab24 | |
| --- | --- |
| Antibody Name | IC50 (nM) |
| Ab3 | 52.67 |
| Ab4 | 71.49 |
| Ab5 | 34.20 |
| Ab21 | 1.13 |
| Ab22 | 0.98 |
| Ab23 | 35.19 |
| Ab24 | 4.09 |
| Chimeric antibody | 1.74 |

[Table 19]

| Antibody Name | 1$^{st}$ Screening | 2$^{nd}$ Screening | 3$^{rd}$ Screening |
| --- | --- | --- | --- |
| | Ag EC50(ng/ml) | Thermal Instability (%) | TF-1 Proliferation Inhibition (nM) |
| Ab1 | 56.4 | 139.9 | |
| Ab2 | 78.16 | 155.4 | |
| Ab3 | 45.95 | 1.3 | 52.67 |
| Ab4 | 57.2 | 57.5 | 71.49 |
| Ab5 | 76.48 | 60.8 | 34.20 |
| Ab6 | 246.37 | 1.5 | |
| Ab7 | N.D | 79.3 | |
| Ab8 | 12350.66 | 97.7 | |
| Ab9 | 92.65 | 160.3 | |

(continued)

| Antibody Name | 1st Screening | 2nd Screening | 3rd Screening |
|---|---|---|---|
| | Ag EC50(ng/ml) | Thermal Instability (%) | TF-1 Proliferation Inhibition (nM) |
| Ab10 | 81.95 | 135.0 | |
| Ab11 | 110.62 | 93.2 | |
| Ab12 | 4386.52 | 75.0 | |
| Ab13 | N.D | 184.7 | |
| Ab14 | 1775.9 | 184.9 | |
| Ab21 | 122.482 | -7.2 | 1.13 |
| Ab22 | 137.368 | -8.1 | 0.98 |
| Ab23 | 107.821 | -14.5 | 35.19 |
| Ab24 | 133.509 | 17.8 | 4.09 |
| Chimeric antibody | 92.84 | 64.8 | 1.74 |

## Conclusion

According to one embodiment of the present disclosure, a histidine-tagged recombinant protein of canine interleukin-13 (cIL-13) was successfully produced. The cIL-13 recombinant protein polypeptide, excluding glycan chains, had a molecular weight of approximately 13 kDa and was expressed as a glycoprotein with excessive N-glycosylation. The cIL-13 exhibited biological activity by inducing TF-1 cell proliferation.

According to one embodiment of the present disclosure, a monoclonal antibody, 32A10, specifically recognizing canine interleukin-13 was successfully developed. Among three subclones, the clone "32A10-#20" showed the highest antigen affinity, with an $EC_{50}$ value of 463 pM. The 32A10 monoclonal antibody inhibited IL-13-dependent TF-1 cell proliferation, with $IC_{50}$ values ranging from 2.9 to 4.2 nM. The 32A10 monoclonal antibody was found to detect the antigen independently of N- and O-glycosylation, recognizing the recombinant protein polypeptide.

According to one embodiment of the present disclosure, two variable region sequences for both heavy and light chains were obtained from the 32A10 monoclonal antibody, and mouse/canine chimeric antibodies were produced. Among the four combinations, the FO-type chimeric antibody showed antigen binding affinity and TF-1 proliferation inhibitory activity similar to the parental clone. Additionally, the FO-type chimeric antibody demonstrated an inhibitory effect on IL-13-induced STAT-6 phosphorylation. Therefore, the final identification of the variable region sequences of 32A10 was F-type for the heavy chain and O-type for the light chain.

The 32A10 monoclonal antibody effectively neutralized cIL-13 activity and inhibited TF-1 cell proliferation with $IC_{50}$ values ranging from 2.2 to 3.3 nM. The strong neutralizing activity of the 32A10 monoclonal antibody against cIL-13 at low concentrations indicated its potential as a therapeutic agent for canine atopic dermatitis. The mouse/canine chimeric antibody with similar activity to the parental antibody confirmed the accuracy of the variable region gene sequences derived from the 32A10 hybrid cell line, and the high purity of the 32A10 chimeric antibody was interpreted as a stable physical property supporting its developability.

According to one embodiment of the present disclosure, the 32A10 chimeric antibody was caninized. In the caninization design process, CDR grafting technology was used to transplant the mouse-derived complementarity-determining regions (CDRs) of the chimeric antibody onto a framework derived from the canine germline. According to one embodiment of the present disclosure, a homology model was first constructed to predict the three-dimensional structure of the chimeric antibody. The sequence of the chimeric antibody was either retained or replaced with a caninized sequence considering Vernier zones and potential post-translational modifications. According to one embodiment of the present disclosure, canine germline genes IGHV1-30*01 and IGHV3-23*01 were selected as templates for the heavy chain variable region, and IGK3-18*01 and IGKV2-10*01 were selected as templates for the light chain variable region through canine gene database searches. Three versions of caninized sequences were designed for each template.

A total of 36 sequences were derived by combining the heavy and light chain sequences. Among 24 named antibodies, Ab1 to Ab14 and Ab21 to Ab24 were successfully obtained. According to one embodiment of the present disclosure, the antigen reactivity, thermal stability, and TF-1 cell proliferation inhibitory activity of the antibodies were evaluated. As a result, caninized antibodies Ab3 to Ab5 and Ab21 to Ab24 were selected as candidates considering productivity and biological activity.

Meanwhile, the above antibodies may be in the form of chimeric antibodies, humanized antibodies, caninized antibodies, bivalent, bispecific molecules, minibodies, domain antibodies, bispecific antibodies, antibody mimetics, diabodies, triabodies, tetrabodies, or fragments thereof, but are not limited thereto.

In the present disclosure, the term "chimeric antibody" refers to an antibody in which the variable region of a murine antibody and the constant region of a canine antibody are recombined, and has significantly improved immune response compared to murine antibodies.

In the present disclosure, the term "humanized antibody" refers to an antibody derived from a non-human species whose protein sequence has been modified to resemble a human antibody variant naturally produced in humans. For example, the humanized antibody may be produced by recombining the murine-derived CDR with a human antibody-derived framework region(FR) to produce a humanized variable region, which is then recombined with the constant region of a preferred human antibody.

In the present disclosure, the antibodies may also be applied in veterinary medicine. For example, a "caninized antibody" refers to an antibody derived from a non-canine species whose protein sequence has been modified to resemble an antibody variant naturally produced in dogs.

Additionally, the present disclosure may provide a pharmaceutical composition for the treatment of immune diseases or cancer comprising the above-described antibody and a pharmaceutically acceptable carrier.

In the present disclosure, the antibody may be in the form of an antibody-drug conjugate (ADC). The "antibody-drug conjugate" refers to a chemical conjugate that links an antibody and a drug without reducing the biological activity of either.

In the present disclosure, the chemical linkage may be a covalent bond. In the present disclosure, the antibody-drug conjugate may be in a form in which the drug is bound to the N-terminal amino acid residues of the heavy and/or light chains of the antibody, more specifically to the $\alpha$-amino group of the N-terminus of the heavy and/or light chains.

The pharmaceutical composition of the present disclosure may be characterized in the form of an injectable formulation and may be intended for administration to at least one of a human or animal.

In the present disclosure, the administration route of the pharmaceutical composition is not limited to, but includes oral, intravenous, intramuscular, intra-arterial, intraosseous, intradural, intracardiac, transdermal, subcutaneous, intraperitoneal, intranasal, enteral, topical, sublingual, or rectal administration; however, non-oral administration is preferred.

In the present disclosure, "non-oral" administration includes subcutaneous, intradermal, intravenous, intramuscular, intra-articular, intra-bursal, sternal, intradural, lesion, and intracranial injection or infusion techniques. The pharmaceutical composition of the present disclosure may also be administered in the form of a suppository for rectal administration.

The pharmaceutical composition of the present disclosure may vary depending on several factors, including the activity of the specific compound used, age, weight, general health, sex, diet, time of administration, route of administration, excretion rate, drug combination, and severity of the disease to be prevented or treated. The dosage of the pharmaceutical composition may also vary depending on the condition, weight, disease severity, drug form, route of administration, and duration, but may be appropriately selected by one skilled in the art.

In the present disclosure, the "immune-related disease" refers to a disease induced by excessive activation and expression of various immune and inflammatory cells, including but not limited to; autoimmune diseases, graft-versus-host disease; organ transplant rejection; asthma; atopy; or acute or chronic inflammatory diseases.

Also, in the present disclosure, the term "autoimmune disease" may include at least one selected from the group consisting of rheumatoid arthritis, systemic sclerosis, systemic lupus erythematosus, atopic dermatitis, psoriasis, alopecia areata, asthma, Crohn's disease, Behçet's disease, Sjögren's syndrome, Guillain-Barré syndrome, chronic thyroiditis, multiple sclerosis, polymyositis, ankylosing spondylitis, fibrositis, and nodular polyarteritis but not limited thereto.

In the present disclosure, "cancer" is a disease characterized by the rapid and uncontrolled growth of mutant cells and may include at least one selected from the group consisting of melanoma, fallopian tube cancer, brain cancer, small intestine cancer, esophageal cancer, lymphoma, gallbladder cancer, hematologic malignancies, thyroid cancer, endocrine cancer, oral cancer, liver cancer, bile duct cancer, colon cancer, rectal cancer, cervical cancer, ovarian cancer, kidney cancer, gastric cancer, duodenal cancer, prostate cancer, breast cancer, brain tumor, lung cancer, anaplastic thyroid cancer, uterine cancer, colorectal cancer, bladder cancer, ureter cancer, pancreatic cancer, bone/soft tissue sarcoma, skin cancer, non-Hodgkin lymphoma, Hodgkin lymphoma, multiple myeloma, leukemia, myelodysplastic syndrome, acute lymphoblastic leukemia, acute myeloid leukemia, chronic lymphocytic leukemia, chronic myeloid leukemia, and solitary myeloma.

The present disclosure may also provide a host cell that produces the antibody.

Host cells used to manufacture antibody drugs may vary depending on the specific antibody and intended use. In general, antibody pharmaceuticals are produced using living cells-such as bacteria, yeast, or mammalian cells-to express the antibody protein, and the choice of host cell can affect the yield, quality, and glycosylation pattern of the final antibody product. For example, host cells may include bacteria such as E. coli, yeast, or mammalian cells such as CHO cells, or HEK cells.

In addition to the selection of host cells, genetic engineering manipulations of the host cells may also be performed to enhance antibody expression and secretion. For instance, insertion of genes encoding chaperone proteins or glycosylation enzymes may enhance the yield and quality of the final antibody product.

The present disclosure may further provide a vector comprising a nucleic acid encoding the antibody.

A vector is a DNA molecule used to deliver foreign genetic material such as a gene encoding an antibody protein into a host cell for the purpose of producing a desired protein. The choice of vector may vary depending on the specific host cell and the purpose of the recombinant protein.

The descriptions of the presented embodiments are provided to enable those skilled in the art to utilize and implement the present disclosure. Various modifications of these embodiments will be apparent to those skilled in the art, and the general principles defined herein may be applied to other embodiments without departing from the scope of the present disclosure. Therefore, the present disclosure is not limited to the embodiments set forth herein but should be interpreted in the broadest scope consistent with the principles and novel features disclosed herein.

[Mode for Carrying Out the Invention]

The relevant details have been described in the best mode for carrying out the invention as above.

[Industrial Applicability]

The present disclosure can be used for the treatment of atopic dermatitis in individuals including dogs.

[Sequence Listing Free Text]

Among the sequences in the sequence listing of this application, Sequence Identification No. 5 is amino acids fewer than four in length and is described separately as follows:
Sequence Identification No. 5
Leu Ala Ser

**Claims**

1. An anti-canis interleukin-13 (anti-cIL-13) antibody or an antigen-binding fragment thereof, which specifically recognizes and neutralizes canis interleukin-13 (cIL-13).

2. The anti-cIL-13 antibody or antigen-binding fragment thereof according to claim 1, comprising a monoclonal antibody.

3. The anti-cIL-13 antibody or antigen-binding fragment thereof according to claim 1, comprising a murine antibody, a chimeric antibody, or a caninized antibody.

4. The anti-cIL-13 antibody or antigen-binding fragment thereof according to claim 1, comprising a heavy chain constant region derived from canine immunoglobulin G (IgG) A, B, C, or D type.

5. The anti-cIL-13 antibody or antigen-binding fragment thereof according to claim 1, comprising a light chain constant region derived from canine kappa or lambda chain.

6. The anti-cIL-13 antibody or antigen-binding fragment thereof according to claim 1, comprising a light chain variable region having at least one amino acid sequence selected from the group consisting of SEQ ID NOs: 12, 23, 24, 25, 26, 27, and 28.

7. The anti-cIL-13 antibody or antigen-binding fragment thereof according to claim 6, wherein the light chain comprises at least one amino acid sequence selected from the group consisting of SEQ ID NOs: 30, 32, 34, 36, 38, 40, 42, 44, 46, 48, 50, 52, 54, 56, 58, 60, 62, 64, 66, 68, 70, 72, 74, 76, and 78.

8. The anti-cIL-13 antibody or antigen-binding fragment thereof according to claim 1, comprising a heavy chain variable region having at least one amino acid sequence selected from the group consisting of SEQ ID NOs: 10, 17, 18, 19, 20, 21, and 22.

9. The anti-cIL-13 antibody or antigen-binding fragment thereof according to claim 8, wherein the heavy chain comprises at least one amino acid sequence selected from the group consisting of SEQ ID NOs: 29, 31, 33, 35, 37, 39, 41, 43, 45, 47, 49, 51, 53, 55, 57, 59, 61, 63, 65, 67, 69, 71, 73, 75, and 77.

10. The anti-cIL-13 antibody or antigen-binding fragment thereof according to claim 1, having at least one characteristic selected from the group consisting of inhibition of TF-1 cell proliferation and inhibition of STAT-6 phosphorylation.

11. The anti-cIL-13 antibody or antigen-binding fragment thereof according to claim 1, comprising:

a heavy chain having an amino acid sequence of SEQ ID NO: 69 or 71 or a variant thereof; and
a light chain having an amino acid sequence of SEQ ID NO: 70 or 72 or a variant thereof.

12. The anti-cIL-13 antibody or antigen-binding fragment thereof according to claim 1, comprising an antibody composed of two light chains and two heavy chains.

13. The anti-cIL-13 antibody or antigen-binding fragment thereof according to claim 1, comprising at least one fragment selected from the group consisting of Fab fragment, Fv fragment, single-chain variable fragment (scFv), diabody, triabody, and tetrabody.

14. A method of treating or preventing a disease in an animal, comprising using the anti-cIL-13 antibody or antigen-binding fragment thereof according to claim 1.

15. The method according to claim 14, wherein the method is applied to the animal, and is for treating of preventing the disease comprising at least one selected from the group consisting of inflammatory diseases, immune diseases, autoimmune diseases, and cancer.

16. The method according to claim 15, wherein the disease comprises atopic dermatitis.

17. The method according to claim 14, wherein the animal comprises a dog.

18. A pharmaceutical composition comprising the anti-cIL-13 antibody or antigen-binding fragment thereof according to claim 1, wherein the pharmaceutical composition is used for treating or preventing a disease in an animal.

19. The pharmaceutical composition according to claim 18, wherein the pharmaceutical composition is formulated in an injectable form.

20. A host cell used for producing an anti-cIL-13 antibody or antigen-binding fragment thereof, which specifically recognizes and neutralizes canine interleukin-13 (cIL-13).

21. The host cell according to claim 20, wherein the host cell is selected from the group consisting of bacteria including Escherichia coli, yeast, CHO cells, and HEK cells.

22. The host cell according to claim 20, comprising a gene encoding a chaperone protein or a glycosylation enzyme as a result of genetic engineering for enhancing antibody expression and secretion.

23. A vector applied to the host cell according to claim 20 for producing the anti-cIL-13 antibody or antigen-binding fragment thereof.

24. The vector according to claim 23, wherein the vector is a DNA molecule comprising a nucleic acid encoding all or part of the anti-cIL-13 antibody.

[Figure 1]

| Canine IL-13 | XhoI | Kozak | Start codon | Signal Peptide | Canine IL-13 | Stop codon | EcoRI |
|---|---|---|---|---|---|---|---|

[Figure 2]

Xhol ——— EcoRI

P_CMV

WPRE

TK pA

Ampicillin

P_SV40

Modified pcDNA3.4

pUC ori

Neomycin

SV40 pA

[Figure 3a]

[Figure 3b]

[Figure 3c]

[Figure 4]

1. Purified recombinant canine IL-13 protein (highly glycosylated)
2. PNGase-treated recombinant canine IL-13 protein
⇐ Deglycosylated recombinant canine IL-13 protein

[Figure 5]

TF1 proliferation

—○— cIL-13 stored at 2-6°C
--□-- Freeze/thaw 1 cycle cIL-13
--△-- Freeze/thaw 3 cycle cIL-13
—▽— R&D cIL-13

[Figure 6]

4: Canine IL-13
5: PNGase F-treated Canine IL-13
6: O-glycosidase-treated Canine IL-13

Detection: 32A10-Biotin/SA-HRP

[Figure 7]

| Heavy Chain | XhoI | Kozak | Start codon | Signal Peptide | 32AH VH | 32AH CH | Stop codon | EcoRI |
|---|---|---|---|---|---|---|---|---|

| Light Chain | XhoI | Kozak | Start codon | Signal Peptide | 32AH VL | 32AH CL | Stop codon | EcoRI |
|---|---|---|---|---|---|---|---|---|

[Figure 8]

[Figure 9]

[Figure 10]

[Figure 11a]

DRCurve_SPL1

Chimeric antibody

DRCurve_SPL2

Ab3

[Figure 11b]

Ab4

Ab5

[Figure 11c]

Ab21

Ab22

[Figure 11d]

Ab23

Ab24

[Figure 12]

[Figure 13a]

Chimeric antibody

Ab3

Ab4

[Figure 13b]

Ab5

Ab21

Ab22

[Figure 13c]

Ab23

Ab24

## INTERNATIONAL SEARCH REPORT

| International application No. |
|---|
| **PCT/KR2024/095498** |

| A. | CLASSIFICATION OF SUBJECT MATTER |
|---|---|
| | **C07K 16/24**(2006.01)i; **A61P 35/00**(2006.01)i; **A61P 37/00**(2006.01)i; **A61K 39/00**(2006.01)i |

According to International Patent Classification (IPC) or to both national classification and IPC

| B. | FIELDS SEARCHED |
|---|---|

Minimum documentation searched (classification system followed by classification symbols)

C07K 16/24(2006.01); C07K 14/52(2006.01); C07K 14/535(2006.01); C07K 16/28(2006.01); C12N 15/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 개속(canis), 인터루킨-13(Interleukin-13), 항체(antibody), 질환(disease), 치료 (treatment)

| C. | DOCUMENTS CONSIDERED TO BE RELEVANT | | |
|---|---|---|---|
| Category* | Citation of document, with indication, where appropriate, of the relevant passages | | Relevant to claim No. |
| X | US 2006-0216786 A1 (SIM, Gek-Kee et al.) 28 September 2006 (2006-09-28) See abstract; and paragraphs [0107], [0112], [0116], [0120]-[0123], [0126]-[0128] and [0253]. | | 1-5,10,12-24 |
| A | | | 6-9,11 |
| A | US 2020-0181258 A1 (VETOQUINOL SA) 11 June 2020 (2020-06-11) See entire document. | | 1-24 |
| A | TANAKA, Kazuaki et al. Association analysis of non-synonymous polymorphisms of interleukin-4 receptor-α and interleukin-13 genes in canine atopic dermatitis. The Journal of Veterinary Medical Science. 15 July 2020 (online publication date), vol. 82, no. 9, pp. 1253-1259. See entire document. | | 1-24 |
| A | WO 2014-152361 A1 (WAKE FOREST UNIVERSITY HEALTH SCIENCES) 25 September 2014 (2014-09-25) See entire document. | | 1-24 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 June 2024** | **21 June 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/095498**

### C. DOCUMENTS CONSIDERED TO BE RELEVANT

| Category* | Citation of document, with indication, where appropriate, of the relevant passages | Relevant to claim No. |
|---|---|---|
| A | YANG, Shumin et al. Canine interleukin-13: molecular cloning of full-length cdna and expression of biologically active recombinant protein. Journal of Interferon and Cytokine Research. 01 September 2000, vol. 20, no. 9, pp. 779-785. See entire document. | 1-24 |

Form PCT/ISA/210 (second sheet) (July 2022)

| INTERNATIONAL SEARCH REPORT | International application No. |
|---|---|
| | **PCT/KR2024/095498** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.   ☑   forming part of the international application as filed.

     b.   ☐   furnished subsequent to the international filing date for the purposes of international search (Rule 13*ter*.1(a)),

         ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2024/095498**

| Patent document cited in search report | | | Publication date (day/month/year) | Patent family member(s) | | | Publication date (day/month/year) |
|---|---|---|---|---|---|---|---|
| US | 2006-0216786 | A1 | 28 September 2006 | EP | 1082432 | A2 | 14 March 2001 |
| | | | | JP | 2002-516104 | A | 04 June 2002 |
| | | | | US | 2002-0127200 | A1 | 12 September 2002 |
| | | | | US | 2003-0099609 | A1 | 29 May 2003 |
| | | | | US | 2003-0143196 | A1 | 31 July 2003 |
| | | | | US | 2004-0191868 | A1 | 30 September 2004 |
| | | | | US | 2007-0009488 | A1 | 11 January 2007 |
| | | | | US | 2007-0141671 | A1 | 21 June 2007 |
| | | | | US | 2009-0111970 | A1 | 30 April 2009 |
| | | | | US | 2010-0285529 | A1 | 11 November 2010 |
| | | | | US | 6471957 | B1 | 29 October 2002 |
| | | | | US | 7078506 | B2 | 18 July 2006 |
| | | | | US | 7183080 | B2 | 27 February 2007 |
| | | | | US | 7427661 | B2 | 23 September 2008 |
| | | | | US | 7629441 | B2 | 08 December 2009 |
| | | | | US | 7780959 | B2 | 24 August 2010 |
| | | | | US | 8263559 | B2 | 11 September 2012 |
| | | | | WO | 01-40313 | A2 | 07 June 2001 |
| | | | | WO | 99-61618 | A2 | 02 December 1999 |
| US | 2020-0181258 | A1 | 11 June 2020 | CN | 110114369 | A | 09 August 2019 |
| | | | | EP | 3526246 | A1 | 21 August 2019 |
| | | | | WO | 2018-073185 | A1 | 26 April 2018 |
| WO | 2014-152361 | A1 | 25 September 2014 | EP | 2970492 | A1 | 20 January 2016 |
| | | | | EP | 2970492 | B1 | 08 May 2019 |
| | | | | US | 10676529 | B2 | 09 June 2020 |
| | | | | US | 11655299 | B2 | 23 May 2023 |
| | | | | US | 2016-0039938 | A1 | 11 February 2016 |
| | | | | US | 2018-0155437 | A1 | 07 June 2018 |
| | | | | US | 2020-0299394 | A1 | 24 September 2020 |
| | | | | US | 2023-0295317 | A1 | 21 September 2023 |
| | | | | US | 9868788 | B2 | 16 January 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190053184


**Non-patent literature cited in the description**

- *Journal of Interferon and Cytokine Research*, 2000, vol. 20, 779-785